# EUROPEAN PATENT APPLICATION

(11) **EP 3 312 283 A1**
(43) Date of publication of application: **25.04.2018**
(21) Application number: 17205393.6
(22) Date of filing: 22.03.2013
(51) Int. Cl.: C12N 15/86, A61K 48/00

(54) **DRUG DELIVERY SYSTEM FOR USE IN THE TREATMENT OR DIAGNOSIS OF NEUROLOGICAL DISORDERS**

(30) Priority: 06.03.2013 WO PCT/EP2013/000656
(62) Divisional of application: 13001491.3
(71) Applicant: Life Science Inkubator Betriebs GmbH & Co. KG, 53175 Bonn (DE)
(72) Inventor: Demina, Victoria, 53175 Bonn (DE); Manninga, Heiko, 37073 Göttingen (DE); Götzke, Armin, 97070 Würzburg (DE); Glassmann, Alexander, 50999 Köln (DE)
(74) Representative: von Renesse, Dorothea

(57) **Abstract**

The invention relates to VLP derived from polyoma virus loaded with a drug (cargo) as a drug delivery system for transporting said drug into the CNS for treatment or diagnosis of a neurological disease, in particular multiple sclerosis, Parkinsons's disease or Alzheimer's disease.

SEQUENCE LISTING

<110>  Life Science Inkubator Betriebs GmbH & Co. KG

<120>  A novel drug delivery system based on JCV-VLP for treatment or
diagnosis of neurological disorders

<130>  54 321 K

<160>  5

<170>  PatentIn version 3.3

<210>  1
<211>  354
<212>  PRT
<213>  JC polyoma virus

<220>
<221>  MISC_FEATURE
<222>  (1)..(354)
<223>  capsid protein VP1

<220>
<221>  MISC_FEATURE
<222>  (10)..(10)
<223>  Xaa is Pro or Arg

<400>  1

Met Ala Pro Thr Lys Arg Lys Gly Glu Xaa Lys Asp Pro Val Gln Val
1               5                   10                  15

Pro Lys Leu Leu Ile Arg Gly Gly Val Glu Val Leu Glu Val Lys Thr
            20                  25                  30

Gly Val Asp Ser Ile Thr Glu Val Glu Cys Phe Leu Thr Pro Glu Met
        35                  40                  45

Gly Asp Pro Asp Glu His Leu Arg Gly Phe Ser Lys Ser Ile Ser Ile
    50                  55                  60

Ser Asp Thr Phe Glu Ser Asp Ser Pro Asn Arg Asp Met Leu Pro Cys
65                  70                  75                  80

Tyr Ser Val Ala Arg Ile Pro Leu Pro Asn Leu Asn Glu Asp Leu Thr
                85                  90                  95

Cys Gly Asn Ile Leu Met Trp Glu Ala Val Thr Leu Lys Thr Glu Val
            100                 105                 110

Ile Gly Val Thr Ser Leu Met Asn Val His Ser Asn Gly Gln Ala Thr
        115                 120                 125

His Asp Asn Gly Ala Gly Lys Pro Val Gln Gly Thr Ser Phe His Phe

130                 135                 140

Phe Ser Val Gly Gly Glu Ala Leu Glu Leu Gln Gly Val Val Phe Asn
145                 150                 155                 160

Tyr Arg Thr Lys Tyr Pro Asp Gly Thr Ile Phe Pro Lys Asn Ala Thr
                165                 170                 175

Val Gln Ser Gln Val Met Asn Thr Glu His Lys Ala Tyr Leu Asp Lys
            180                 185                 190

Asn Lys Ala Tyr Pro Val Glu Cys Trp Val Pro Asp Pro Thr Arg Asn
        195                 200                 205

Glu Asn Thr Arg Tyr Phe Gly Thr Leu Thr Gly Gly Glu Asn Val Pro
    210                 215                 220

Pro Val Leu His Ile Thr Asn Thr Ala Thr Thr Val Leu Leu Asp Glu
225                 230                 235                 240

Phe Gly Val Gly Pro Leu Cys Lys Gly Asp Asn Leu Tyr Leu Ser Ala
                245                 250                 255

Val Asp Val Cys Gly Met Phe Thr Asn Arg Ser Gly Ser Gln Gln Trp
            260                 265                 270

Arg Gly Leu Ser Arg Tyr Phe Lys Val Gln Leu Arg Lys Arg Arg Val
        275                 280                 285

Lys Asn Pro Tyr Pro Ile Ser Phe Leu Leu Thr Asp Leu Ile Asn Arg
    290                 295                 300

Arg Thr Pro Arg Val Asp Gly Gln Pro Met Tyr Gly Met Asp Ala Gln
305                 310                 315                 320

Val Glu Glu Val Arg Val Phe Glu Gly Thr Glu Glu Leu Pro Gly Asp
                325                 330                 335

Pro Asp Met Met Arg Tyr Val Asp Lys Tyr Gly Gln Leu Gln Thr Lys
            340                 345                 350

Met Leu

<210>  2
<211>  1065
<212>  DNA
<213>  JC virus

<220>
<221> MISC_FEATURE
<222> (1)..(1065)
<223> capsid protein VP1

<400> 2
atggctccca ccaagcgcaa gggcgagcsc aaggaycccg tgcaagtgcc caagctgctg 60

atccgtggtg gtgtcgaggt gctggaagtc aagaccggcg tggactccat taccgaggtg 120

gagtgcttcc tcacccccga gatgggtgac cctgacgagc acctgagggg cttctccaag 180

tccatctcca tctccgacac cttcgagtcc gactccccca accgtgacat gctgccctgc 240

tactccgtgg ctcgtatccc cctgcccaac ctgaacgagg acctgacttg cggcaacatc 300

ctgatgtggg aggctgtgac cctcaagacc gaggtcatcg gcgtgacttc cctgatgaac 360

gtgcactcca acggccaggc tacccacgac aacggtgctg gcaagcccgt gcagggaacc 420

tccttccact tcttctccgt gggtggcgag gctctggaac tccagggcgt ggtgttcaac 480

taccgtacca agtaccccga cggcaccatc ttccccaaga acgctactgt gcagtcccaa 540

gtgatgaaca ccgagcacaa ggcttacctg gacaagaaca aggcctaccc cgtggagtgc 600

tgggtgcccg accccacccg taacgagaac acccgttact tcggcaccct gaccggtgga 660

gagaacgtgc cccccgtgct gcacatcacc aacaccgcta ccaccgtgct gctggacgag 720

ttcggtgtcg gtcccctgtg caagggcgac aacctgtacc tgtccgctgt ggacgtgtgc 780

ggcatgttca ccaaccgttc cggttcccag cagtggcgtg gcctgtcccg ctacttcaag 840

gtgcagctgc gcaagcgtcg tgtgaagaac ccctacccta tctccttcct gctgaccgac 900

ctgatcaacc gtcgtacccc tcgtgtggac ggccagccca tgtacggcat ggacgctcag 960

gtggaagagg tccgcgtgtt cgagggcacc gaggaattgc ccggcgaccc cgacatgatg 1020

cgttacgtgg acaagtacgg ccagctccag accaagatgc tgtaa 1065

<210> 3
<211> 344
<212> PRT
<213> JC virus

<220>
<221> MISC_FEATURE
<222> (1)..(344)
<223> capsid protein VP2

<400> 3

Met Gly Ala Ala Leu Ala Leu Leu Gly Asp Leu Val Ala Thr Val Ser
1               5                   10                  15

Glu Ala Ala Ala Ala Thr Gly Phe Ser Val Ala Glu Ile Ala Ala Gly
            20                  25                  30

Glu Ala Ala Ala Thr Ile Glu Val Glu Ile Ala Ser Leu Ala Thr Val
        35                  40                  45

Glu Gly Ile Thr Ser Thr Ser Glu Ala Ile Ala Ala Ile Gly Leu Thr
    50                  55                  60

Pro Glu Thr Tyr Ala Val Ile Thr Gly Ala Pro Gly Ala Val Ala Gly
65                  70                  75                  80

Phe Ala Ala Leu Val Gln Thr Val Thr Gly Gly Ser Ala Ile Ala Gln
                85                  90                  95

Leu Gly Tyr Arg Phe Phe Ala Asp Trp Asp His Lys Val Ser Thr Val
            100                 105                 110

Gly Leu Phe Gln Gln Pro Ala Met Ala Leu Gln Leu Phe Asn Pro Glu
        115                 120                 125

Asp Tyr Tyr Asp Ile Leu Phe Pro Gly Val Asn Ala Phe Val Asn Asn
    130                 135                 140

Ile His Tyr Leu Asp Pro Arg His Trp Gly Pro Ser Leu Phe Ser Thr
145                 150                 155                 160

Ile Ser Gln Ala Phe Trp Asn Leu Val Arg Asp Asp Leu Pro Ser Leu
                165                 170                 175

Thr Ser Gln Glu Ile Gln Arg Arg Thr Gln Lys Leu Phe Val Glu Thr
            180                 185                 190

Leu Ala Arg Phe Leu Glu Glu Thr Thr Trp Ala Ile Val Asn Ser Pro
        195                 200                 205

Val Asn Leu Tyr Asn Tyr Ile Ser Asp Tyr Tyr Ser Arg Leu Ser Pro
    210                 215                 220

Val Arg Pro Ser Met Val Arg Gln Val Ala Gln Arg Glu Gly Thr Tyr
225                 230                 235                 240

Ile Ser Phe Gly His Ser Tyr Thr Gln Ser Ile Asp Asn Ala Asp Ser
                245                 250                 255

Ile Gln Glu Val Thr Gln Arg Leu Asp Leu Lys Asn Pro Asn Val Gln
            260                 265                 270

Ser Gly Glu Phe Ile Glu Lys Ser Phe Ala Pro Gly Gly Ala Asn Gln
        275                 280                 285

Arg Ser Ala Pro Gln Trp Met Leu Pro Leu Leu Leu Gly Leu Tyr Gly
    290                 295                 300

Thr Val Thr Pro Ala Leu Glu Ala Tyr Glu Asp Gly Pro Asn Lys Lys
305                 310                 315                 320

Lys Arg Arg Lys Glu Gly Pro Arg Ala Ser Ser Lys Thr Ser Tyr Lys
                325                 330                 335

Arg Arg Ser Arg Ser Ser Arg Ser
            340

<210>  4
<211>  1032
<212>  DNA
<213>  JC virus

<220>
<221>  MISC_FEATURE
<222>  (1)..(1032)
<223>  capsid protein VP2

<400>  4
atgggtgccg cacttgcact tttgggggac ctagttgcta ctgtttctga ggctgctgct      60

gccacaggat tttcagtagc tgaaattgct gctggagagg ctgctgctac tatagaagtt     120

gaaattgcat cccttgctac tgtagagggg attacaagta cctctgaggc tatagctgca     180

ataggcctta ctcctgaaac atatgctgta attactggag ctccgggggc tgtagctggg     240

tttgctgcat tggttcaaac tgtaactggt ggtagtgcta ttgctcagtt gggatataga     300

ttttttgctg actgggatca taaagtttca acagttgggc tttttcagca gccagctatg     360

gctttacagt tatttaatcc agaagactac tatgatattt tatttcctgg agtgaatgcc     420

tttgttaaca atattcacta tttagatcct agacattggg gcccttcttt gttctccaca     480

atctcccagg ctttttggaa tcttgttaga gatgatttgc catctttaac atctcaggaa     540

attcaaagaa gaacccaaaa actatttgtt gaaactttag caaggttttt ggaagaaact     600

acttgggcaa tagttaattc accagttaac ttatataatt atatttcaga ctattattct     660

agattgtctc cagttaggcc ctctatggta aggcaggttg cccaaaggga gggaacctat     720

atttcctttg gccactcata cacccaaagt atagataatg cagacagcat tcaagaagtt     780

acccaaaggc tagatttaaa aaacccaaat gtgcaatctg gtgaatttat agagaaaagt     840

tttgcaccag gaggtgcaaa tcaaagatct gctcctcaat ggatgttgcc tttactttta     900

gggttgtacg ggactgtaac acctgctctt gaagcatatg aagatggccc caacaaaaag     960

aaaaggagaa aggaaggacc ccgtgcaagt tccaaaactt cttataagag gaggagtaga    1020

agttctagaa gt                                                          1032

<210>  5
<211>  8
<212>  PRT
<213>  Artificial

<220>
<223>  nuclear localization signal

<400>  5

Cys Pro Gly Ala Ala Pro Lys Lys
1               5

## Description

### FIELD OF THE INVENTION

The invention relates to the use of virus like particles (VLP) of the type of human polyoma virus for use as drug delivery system for the treatment or diagnosis of neurological disorders.

### BACKGROUND OF THE INVENTION

One of the major challenges in treatment or diagnosis of neurological disorders is the drug delivery. Drug delivery to a selected site of action (e.g. the CNS neurons for Alzheimer's or Parkinson's disease.) is called drug targeting. By drug targeting an increase of drug concentration at this specific site becomes possible even with a systemic application of the drug. Additionally, contrary to the ordinary systemic application, the rest of the body is not or only to a lower extend exposed to the drug. This leads to a reduced risk of adverse side effects and can allow a higher dosing of the drug. Furthermore, very toxic drugs (e.g. cytotoxic agents used in cancer therapeutics) may be applicable to humans for the first time, since their toxic side effects are minimized by the drug delivery system. In some cases, another advantage of drug targeting is the prevention of early inactivation (metabolism), unwanted adsorption, excretion or unwanted modification of the drug, since the drug is protected by the mode of delivery.

Drug delivery into the central nervous system (CNS), in particular into the specific cells such as oligodendrocytes, microglia or neurons, is a great challenge, since the active ingredients at first have to cross the blood-brain barrier and then have to reach the target cells and possibly also enter these cells.

The blood-brain barrier (BBB) is formed by the endothelium of the capillaries. These endothelial cells are tightly connected by so-called "tight junctions" to each other and therewith prevent the entry of substances above a certain molecular weight size into the CNS. The blood-brain barrier thus serves as an effective protective barrier. It guarantees on the one hand the supply of nutrients to the CNS and, on the other hand, enables the removal of metabolic products out of the CNS.

The main concern is the transport of hydrophilic substances across the blood-brain barrier. Nearly 95% of all effective *in vitro* drug candidates are not able to pass the BBB in pharmacologically active concentrations. Therefore, to reach adequate pharmacologically concentrations of the drug within the CNS, the treatment of many CNS diseases such as brain tumors or CNS infections require high plasma concentrations of the drug. This includes the risk of adverse side effects. In pharmaceutical research therefore ways are sought to improve the transport of drugs, in particular hydrophilic agents, over the BBB into the CNS.

Some approaches use lipophilic particles, which allow a receptor-mediated transport across the blood-brain barrier. For this purpose, for example, in particular transport systems of nanoparticles have been developed. Nanoparticles are usually composed of polymers and have a size of 10-1000 nm. Mostly they are surface modified.

These nanoparticles often face the problem that they are exported from the CNS by efflux pumps which are expressed in the BBB. Furthermore, it has been shown that they affect the integrity of the BBB (Rempe et al., Biochem Bioph Res Comm 2011, 406 (1): 64-69). Thus, the protective function of the BBB is put under risk, including the risk of side effects by the entry of unwanted substances or infective entities into the CNS. This substantial disadvantage is critical for the clinical application of nanoparticles as drug delivery systems.

Other possible drug delivery systems are under discussion. For example, it is known that pseudocapsids from VP1 protein of murine polyomavirus associated with β-galactosidase-coding DNA can be used to deliver that DNA into the brain after intravenous administration, leading to the expression of β-galactosidase in the brain (Krauzewicz et al. Gene Therapy 2000, 7: 1094-1102; see also WO 98/48841 A1). However, these observations do not provide sound insights into the suitability of VLP, in particular VLP derived from human polyoma virus and loaded with a cargo, as a drug delivery system for the CNS.

It is therefore an objective of the present invention to provide a drug delivery system for the treatment and diagnosis of neurological disorders.

It is a particular objective of the present invention to provide a drug delivery system for targeting cells of the CNS, which then allow the targeted transport of drugs for the treatment or diagnosis of neurological disorders. It is understood that by a "drug" all substances with therapeutic and/or diagnostic purposes are meant.

### SUMMARY OF THE INVENTION

The invention solves this objective by the provision of VLPs derived from human polyoma virus as a drug delivery system, which in particular serve for the drug transport to and/or into microglia cells or neurons of the CNS. This drug delivery system allows a targeting of the drug substance to CNS cells which are relevant for the treatment or diagnosis of neurological disorders, such as morbus Parkinson or morbus Alzheimer.

The inventors have found in a BBB model based on primary porcine endothelial cells that VLP of human JCV origin loaded with a nucleic acid coding for a reporter protein can cross the BBB and deliver the nucleic acid to distally located cells, where the nucleic acid is expressed. This model is considered representative of the physiologically intact BBB *in vivo.* Therefore it shows that the VLP, and not just the active substance alone, can cross the BBB and enter into the CNS. Within the CNS the VLP can target microglia cells, oligodendrocytes and/or neurons.

By these findings the inventors could further demonstrate that the crossing of the loaded VLP does neither require nor lead to a loss of integrity or increased permeability of the BBB. Hence, the integrity of the BBB is not substantially affected by the VLP. Thus VLP can be used as drug delivery system into the CNS without the substantial risk of adverse side effects due to the entry of unwanted substances or infectious entities into the CNS.

Furthermore, the intravenous administration of said VLPs in mice with an intact blood brain barrier resulted in a cellular localization of the VP1 protein and the cargo within the CNS, thereby proving the feasibility of these VLPs for a cellular targeting within the CNS. Importantly, the use of a luciferase encoding expression vector as cargo resulted in the successful cellular localization of the Luciferase protein and detection of Luciferase activity. Hence the cargo was not degraded within the cell (i.e. by delivery to lysosomes) but was expressed to yield the desired functionally active protein.

Finally, the inventors were able to identify these CNS target cells as neurons, microglia cells and oligodendrocytes.

Accordingly, VLP derived from polyoma virus and loaded with a cargo substance is suitable as a drug delivery system to and/or into CNS cells preferably into microglica, neurons or oligodendrocytes. Especially the delivery of the cargo to microglia cells and neurons represents a surprising finding, since these cells are not known to be infected by the JCV virus, which is the virus from which the VP1 proteins of the VLP were derived.

In one aspect of the invention, VLP derived from polyoma virus and loaded with a cargo are used to cross the BBB together with the cargo. Importantly, the crossing of the BBB by the VLP enables the VLP to exhibit its function of targeting specific cell populations within the brain, i.e. deliver the cargo to targeted cells. In the context of the invention said delivery comprises a delivery to and/or into the targeted cells. Hence according to the invention a functional VLP crosses the BBB together with the cargo.

The cargo, preferably an active substance, is preferably fully encapsulated in the hull of the VLP. This however does not exclude that the VLP additionally can be associated with the active substance, e.g. by way of binding of the active substance to outside structures of the hull. Some molecules of the active substance might even be incompletely encapsulated within the hull. However, at least a part of the molecules of the active substance is fully encapsulated in the hull.

In the context of the invention the cargo of the VLP can be delivered to and/or into the target CNS cell, in particular microglia, oligodendrocytes and/or neurons, which reflects the following two targeting situations:
1.) A delivery within the target cell, i.e. a delivery, whereby the cargo of the VLP without or with the VLP is delivered across the plasma membrane of the target cell into the cytoplasma, intracellular compartments such as endosome, or a target compartment such as the cell nucleus, thereby representing a delivery into the cell. This type of delivery encompasses a delivery of the VLP across the plasma membrane whereby the cargo is released from the infected cell to exert its function on the outer side of the plasma membrane (e.g. at membrane receptors) or at adjacent cells or tissue structures.
2.) A delivery to the surface of the target cell, i.e. a delivery, whereby the VLP can e.g. dock at the target cell and delivers its cargo to the outside of said cell, possibly including a potential binding of the cargo to the cell membrane or membrane protein. In the context of the present invention this delivery is understood as a delivery to the cell.

In a preferred embodiment of the invention the VLP provides a delivery of the cargo into the target cell, particular into an oligodendrocyte, a microglia cell or into a neuron.

Hence according to the invention a composition of VLP derived from polyoma virus and an active substance is provided as a drug delivery system, wherein at least 1%, at least 2%, at least 3%, at least 5%, at least 7%, at least 10%, at least 15%, at least 20%, at least 25%, at least 30%, at least 40%, at least 50%, at least 60%, more preferably at least 70%, at least 75%, at least 80%, at least 85%, at least 90%, at least 95%, at least 97%, at least 98%, at least 99% or 100% of the total amount of the active substance (cargo) is encapsulated in the hull of the VLP.

In a most preferred embodiment of the invention the VLPs of the composition are non-aggregated. Non-aggregated means that the VLPs are able to form separated particles, when being suspended in water.

In one aspect of the invention thus a drug delivery system for the CNS is provided which allows a co-transport of the VLP and the active substance. The transport can be either active or passive. Interestingly the inventors furthermore found that the VLP are not or not substantially removed from the CNS by efflux transporters of the BBB.

The VLP as of the invention do not require a surface treatment or the use of additives in order to efficiently cross the BBB and enter into the CNS. Hence in one aspect of the invention a drug delivery system is provided with particulate structures without any further surface treatment or modification and without the use of additives. The drug delivery system preferably is cell-free.

As outlined above, according to the invention, the VLP are used for drug delivery into the CNS, comprising the spinal cord and the brain and in particular into the brain. The delivery into the brain can enable a drug targeting to and/or into specific cells which allows the diagnosis and treatment of specific neurological diseases.

In one aspect of the invention, this targeting comprises the delivery to oligodendrocytes, thereby taking advantage of the natural tropism of the polyoma virus (specifically of JCV). Based on this cellular target, VLPs of the invention allow the diagnosis and treatment of neurological disorders associated with or caused by oligodendrocytes. In a preferred embodiment the VLPs allow the diagnosis or treatment of multiple sclerosis, which represents a demyelination disease with a key role of oligodendrocytes.

In a further aspect of the invention, this targeting comprises the delivery to microglia cells, which are not known as naturally target cells of polyoma virus (specifically of JCV). Based on this cellular target, VLPs of the invention allow the diagnosis and treatment of neurological disorders associated with or caused by microglia cells. Hence, in a preferred embodiment, the VLPs allow the diagnosis or treatment of Alzheimer's disease, which represents a neurodegenerative disease in which various inflammatory processes and cytokines play a role. Furthermore, this targeting profile enables the diagnosis or treatment of Parkinson's disease. Although the disease mechanisms that cause Parkinson's disease are still not fully understood, it is believed that the progressive nature of Parkinson's disease is characterized by chronic inflammation-induced neurodegeneration of dopamine-producing neurons within the substantia nigra and striatum. It is now well documented that microglial activation results in the loss of dopaminergic neurons (DA-neurons) in patients with Parkinson's disease (M. L. Block and J. S. Hong, "Chronic microglial activation and progressive dopaminergic neurotoxicity," Biochemical Society Transactions, 35(5): 1127-1132, 2007).

In a particular preferred embodiment the invention relates to a targeting comprising the delivery into or to CNS neurons. Neurons in the CNS are not known as natural target cells of human polyoma virus (specifically of JCV). Based on this cellular target, VLPs of the invention allow the diagnosis and treatment of neurological disorders associated with or caused by neurons. Hence, in a preferred embodiment, the VLPs allow the diagnosis or treatment of Parkinson's disease.

### DETAILED DESCRIPTION OF THE INVENTION

The structural proteins of the VLP, in particular the VP1 (but also the VP2 and/or the VP3), are identical to or are derived from structural proteins from the viruses of the family of polyomaviridae which includes the three genera orthopolyomavirus (type species SV40), wukipolyomavirus (type species KI polyomavirus) and avipolyomavirus.

Non-limiting examples for viruses of the Polyoma family are B-lymphotropic polyomavirus (formerly known as African green monkey polyomavirus, AGMPyV) (LPyV), Baboon polyomavirus 1 (SA12), Bat polyomavirus (formerly known as Myotis polyomavirus, MyPyV; BatPyV) BK polyomavirus (BKPyV), Bornean orang-utan polyomavirus (OraPyV1), Bovine polyomavirus (BPyV), California sea lion polyomavirus (SLPyV), Hamster polyomavirus (HaPyV), JC polyomavirus (JCPyV), Merkel Cell polyomavirus (MCPyV), Murine pneumotropic virus (formerly known as Kilham strain of polyomavirus, Kilham virus, K virus; MPtV), Murine polyomavirus (MPyV), Simian virus 40 (formerly known as Simian vacuolating virus 40; SV40), Squirrel monkey polyomavirus (SqPyV), Sumatran orang-utan polyomavirus (OraPyV2), Trichodysplasia spinuolsa-associated polyomavirus (TSPyV), Human polyomavirus 6 (HPyV6), Human polyomavirus 7 (HPyV7), KI polyomavirus (formerly known as Karolinska Institute polyomavirus, KIPyV), WU polyomavirus (formerly known as Washington University polyomavirus, (WUPyV), Avian polyomavirus (formerly known as Budgerigar Fledgling disease polyomavirus, BFPyV, APyV), Canary polyomavirus (CaPyV), Crow polyomavirus (CPyV), Finch polyomavirus (FPyV), Goose Hemorrhagic polyomavirus (GHPyV), Athymic rat polyomavirus (RatPyV), Baboon polyomavirus 2 (BPyV2), Cynomolgus polyomavirus (CyPV), Gorilla gorilla gorilla polyomavirus 1 (GggPyV1), Human polyomavirus 9 (HPyV9), Trichodysplasia spinulosa-associated polyomavirus (TSV)Mastomys polyomavirus (multimammate mouse - Mastomys species), Pan troglodytes verus polyomavirus 1 a (PtvPyV1a), Pan troglodytes verus polyomavirus 2c (PtvPyV2c), Rabbit kidney vacuolating virus (RKV).

In a preferred embodiment of the invention, the VLP is derived from a human polyoma virus comprising Human polyomavirus 6 (HPyV6), Human polyomavirus 7 (HPyV7), Human polyomavirus 9 (HPyV9), BK polyomavirus (BKPyV), JC polyomavirus (JCPyV), Merkel Cell polyomavirus (MCPyV), KI polyomavirus (formerly known as Karolinska Institute polyomavirus, KIPyV), WU polyomavirus (formerly known as Washington University polyomavirus, (WUPyV), Trichodysplasia spinulosa-associated polyomavirus (TSV), human polyoma virus 10 (HPyV10), MW polyomavirus and MX polyomavirus.

In a more preferred embodiment of the invention, the VLP is derived from the human polyoma virus JCV.

In the context of the present invention the term "derived from human polyoma virus" refers to a VLP with structural proteins that can be isolated or extracted from viruses or which can be generated by recombinant expression of a polyoma structural protein or a modified form of said structural protein.

A virus-like particle (VLP) derived from polyoma virus, in particular from human polyoma virus and more particular from the virus JCV, is used for drug delivery according to the present invention preferably comprising at least one VP1. The VLP is preferably composed of a hull build up of VP1 assembled into pentameric structures. Preferably, the VLP is composed of several VP1, in particular several VP1 pentamers, especially 72 VP1 pentamers. However, the VLP may optionally comprise further molecules incorporated into the hull. The structure molecules assembling the VLP can either be identical to the native polyoma virus proteins or can be modified in order to optimize the VLP characteristics.

In a preferred embodiment, the structural protein is identical to the native polyoma virus protein or consists essentially of the native polyoma virus protein. According to the invention a structural protein is said to consist essentially of the native polyoma protein if the optional modifications do not change the tropism of the VLP compared of the tropism of the native polyoma virus. This comprises modifications such as the presence of a nuclear localization signal.

In another embodiment the native polyoma virus protein can be modified in order to optimize the VLP with regard to its production, its cellular targeting profile and specificity and its intracellular targeting profile and specificity.

In a preferred embodiment the modification of the polyoma virus protein changes the cellular target of the VLP in a way that a different cell type of the CNS is addressed by the VLP of the invention. The polyoma virus protein can be modified by insertion, deletion or substitution of one or more amino acids. Furthermore, the virus protein can be linked with another protein or peptide by e.g. forming a fusion protein. The protein could be further modified post-trasnlationally. Typical modifications are post-translational modifications that include lipids, acetate groups, phosphate groups, or carbohydrates. The protein could also be chemically modified with e.g. biotin groups.

The VLP according to the invention can furthermore have one or several additional heterologous proteins in the capsid structure, i.e. proteins which are not identical to or derived from a polyoma virus protein. This means that a heterologous protein can be anchored in the capsid structure, at least part of this protein being preferably accessible from outside. In principle all proteins are suitable as this heterologous protein as long as they can be incorporated into the capsid structure and do not interfere substantially with the assembly of the VLP. For example it may be desirable to provide the VLP with an antigenic determinant by means of the heterologous protein that can be detected by a specific antibody. On the other hand the heterologous protein can be a binding partner for a cell surface receptor which enables an interaction of the VLP with a specific class or subclass of cells which carry the corresponding receptor. After binding to the receptor such an interaction is for example the triggering of a particular signal or the internalization of the VLP.

Furthermore, the VLP according to the present invention further comprises a cargo load. In a particular preferred embodiment of the invention the major part of the total amount of the cargo is fully incorporated into the hull. To describe the full encapsulation of the cargo molecule by the VLP the term "loaded" is used. Hence a "loaded VLP" is a VLP with a fully encapsulated cargo.

Said cargo load may be any molecule or composition fitting inside the space surrounded by the hull. Preferably, the cargo load is a CNS-active agent, a detectable agent such as a radionuclide, a protein, a peptide or a nucleic acid, in particular selected from the group consisting of nucleic acids encoding a desired protein such as mRNA, cDNA, a plasmid or vector, inhibitory nucleic acids such as siRNA or miRNA and nucleic acids having catalytic activity such as a ribozyme. The cargo load is sometimes referred hereinafter as the "active substance", "drug", "drug substance" or "active ingredient". As long as not otherwise explicitly mentioned these terms are used as synonyms. Notably, in the context of the invention the term "drug" as a cargo load comprises also substances which can be used for diagnostic purposes.

In a further embodiment the VLP according to the invention is loaded with a drug which selected from the group consisting of monoclonal antibodies, antipsychotic drugs, analgesic drugs, thrombolytics, antidepressants, immunmodulators, immunosuppressants, acetylcholinesterase inhibitors, glutamate receptor antagonists or modulators, such as NMDA receptor antagonists, psychostimulants, anti-dementia drugs, anxiolytic drugs, nootropic drugs, metabolic enhancers, metabolic modulators, neuroprotective drugs, and anticonsulvants.

In one embodiment, the VLP according to the invention is loaded with a drug selected from the group consisting of ABT354, ABT363, ABT436, ABT560, ADX48621, amantadine, AMG 579, AMG 747, Anti Lingo, ARX 424, ASP0777, ASP2905, ATI355, ATX-MS-1467, AZD1446, beta Secretase Inhibitor, BIIB037 (BART), BMS241027, CS-0777, CX1010, elpetrigine, FK949E, GRT-6006, GSK1223249, GSK356278, LuAF11167, MK-4409, MK-7288, MK-8931, naluzotan, Neublastin, ordopidine, PI-2301, R1662, R7129, R7314, SAR127963, SAR228810, SB623, SEP-225441, SEP-228432, SPN802, SUN13837, T2007, TAK-065, TAK-937, TD-8954, TD-9855, UCB0942, V950, PF-04427429, PF-04531083, PF-04958242, PF-05180999, PF-05236812, ABT110, ABT126, ABT288, ABT384, ABT639, ABT652, ABT894, ACC-001, AFQ056, AGN209323, ALKS 33, AZD5213, AZD6765, BAF312, BGG492, BI 44370 TA, BMS708163, BMS820836, BMS927711, BMS954561, bupivacaine, CAD106, caprylic triglyceride, creatine PD-02, CX-717, Davunetide/AL-108, Debio-9902, DS-5565, EHT-0202, ELND-005, fipamezole (BVF025, JP1730), Firategrast, fulranumab, Ganaxolone, GRT-6005, GSK249320, GSK561679, GSK649868, GSK742457, GSK933776, HF0220, HT-0712, Huperzine A, ICA-105665, ispronicline, JNJ-26489112, Lu AA24530, LuAE58054, LY2062430, MCC-257, MEM-3454, methylthioninium chloride, MK-0249, MK-3697, MK-6096, naldemedine, NIC002, NIC5-15, NP002, ONO-4641, OPC-249, PBT2, PF-02545920, PF-03049423, PF-03654746, PF-04360365, PF-04494700 (TTP488), PF-05089771, PF-05236806, plasmid encoding aromatic L-amino acid decarboxylase, PRX-03140, R1578, R7090, R7412, radiprodil, S-33138, S-90098, SAM-531, SAR110894, SAR292833, SB-742457, SEP-225289, SEP-227162, simvastatin, SSR-180711, TA-5538, TAK-428, talampanel, tedatioxetine, TV-1102, VX-765, YKP3089, ABT957, ALKS 36, camor, MRZ-8676, alemtuzumab, AVP-923, brexpiprazole, brivaracetam, capsaicin, cariprazine, carisbamate, daclizumab, desmoteplase, dexpramipexole, dimebolin hydrochloride, GSK587124, idebenone, laquinimod, latrepirdine, levadex, levomilnacipran, LY-2140023, LY-2216684 (NERI IV), LY450139, mavoglurant, mecobalamin, midazolam, nabiximols, naloxegol, ocrelizumab, preladenant, R1678, rosiglitazone XR, safinamide, solanezumab, suvorexant, vortioxetine, zicronapine, 4-aminopyridine, acamprosate, ademetionine, agomelatine, almorexant, alprazolam, amisulpride, amitriptyline, amitriptylinoxide, amphetamine, anatibant, apomorphine, aripiprazole, armodafinil, asenapine, asenapine, atomoxetine, AZD2066, AZD2423, AZD3839, bapineuzumab, benserazide, betahistine, bifeprunox, blonanserin, bromazepam, brotizolam, buprenorphine, bupropion, buspirone, carbamazepine, carbidopa, Carpipramine, CDP323, celecoxib, citalopram, cladribine, clobazam, clomipramine, clonazepam, clonidine HCL, clozapine, dalfampridine, desflurane, desipramine, desvenlafaxine, dexketoprofen trometamol, dextromethorphan, quinidine sulfate, diazepam, dihydroergotamine mesylate, dimethyl fumarate, sodium valproate, valproic acid, donepezil, dosulepin, doxepin, droxidopa, duloxetine, edaravone, eletriptan, entacapone, escitalopram, eslicarbazepine acetate, eszopiclone, etizolam, ezogabine, fentanyl, fingolimod, fluoxetine, flupirtine, fluvoxamine, fluvoxamine, fosphenytoin, fospropofol, frovatriptan, gabapentin, galantamine, glatiramer acetate, glycopyrrolate, guanfacine, ghrelin, hyaluronic acid, hydrocodone, hydromorphone, hydroxyzine, ibandronic acid, iloperidone, imipramine, indometasin, interferon beta-1a, iproniazid, isocarboxazid, istradefylline, ketorolac tromethamine, lacosamide, lamotrigine, levetiracetam, levodopa, lisdexamfetamine, lofepramine, lormetazepam, Lu02-750, LuAA24493, LuAA34893, LuAA39959, LuAE04621, lurasidone, maprotiline, MEDI-578, memantine, methylphenidate, mianserin, milnacipran, mirtazapine, mitoxantrone, moclobemide, modafinil, morphine, nalmefene, naltrexone, naproxen, naratriptan, natalizumab, nefazodone, nortriptyline, novantrone, olesoxime, olanzapine, oxcarbazepine, oxitriptan, oxycodone, paliperidone, paracetamol, pardoprunox, paroxetine, perampanel, pergolide, perospirone, phenelzine, phenytoin, piracetam, piribedil, pozanicline tartrate, pramipexole, pregabalin, propofol, protriptyline, quazepam, quetiapine, ramelteon, rasagiline, reboxetine, retigabine, rilmazafone, riluzole, risperidone, rivastigmine, rizatriptan, rocuronium bromide, ropinirole, rotigotine, rufinamide, selegiline, sertindole, sertraline, setiptiline, sevoflurane, SLV334, sodium oxybate, sugammadex, sulbutiamine, sulpiride, sumatriptan, tafamidis meglumine, taltireline, tandospirone, tapentadol, TC-5214, TC-5619, telcagepant, teriflunomide, tetrabenazine, tianeptine, tiapride, tissue plasminogen inhibitor (tPA), tizanidine, topiramate, tramadol, tranylcypromine, trazodone, trimipramine, tryptophan, varenicline, venlafaxine, vigabatrin, viloxazine, ziprasidone, zolmitriptan, zolpidem, and zonisamide a dihydropyridin calcium channel blocker such as amlodipine, aranidipine, azelnidipine, barnidipine, benidipine, cilnidipine, clevidipine, cronidipine, darodipine, dexniguldipine, efonidipine, elnadipine, elgodipine, felodipine, flordipine, furnidipine, iganidipine, isradipine, lacidipine, lemildipine, lercanidipine, manidipine, mesuldipine, nicardipine, nifedipine, niguldipine, nimodipine, niludipine, nilvadipine, nisoldipine, nitrendipine, olradipine, oxodipine, palonidipine, pranidipine, sagandipine, sornidipine, teludipine, tiamdipine, trombodipine, watanidipine, (aripiprazole), ABT089 (pozanicline), ABT894 (sofnicline), aricept (donepezil), AZ3480/TC1734 (ispronicline), AZD1446, AZD5213, bavisant/JNJ31001074, guanfacine, ampakine, droxidopa (L-dihydroxyphenyl-serine), DU28853/PGI256 (eltoprazine), KP106 (prodrug of D-amphetamine), lobeline, LY2216684 (edivoxetine), MG01CI (metadoxine), MK0249, ND0801, NERI IV, methylphenidate, OPC-34712, PD9475 (betahistine), PF3654746, SPN811, SPN812, TC-5619, and zalvari/SPN810 (molindone), ALS02 (creatine monohydrate), E0302 (mecabalamin), CK-201357 (tirasemtiv), ampakine (CX-516), arbaclofen (STX-209), carbetocin, D-cycloserine, lenalidomide, methylphenidate, oxytocin, sapropterin, (Kuvan), STX107, synthetic human secretin (RG1068), synthetic porcine secretin, ChiRhoClin, AAB-003, AAD-2004, ABT-126, ABT-288, ABT-384, ABT-560, ACC-001 (vanutide cridificar), ACI-24, ACI-91 (pirenzepine), acrescent (memantine/donepezil), actos (pioglitazone), AD-01, AD02, AD03, albutein + flebogamma (albumin/immunoglobulin), ANAVEX-2- 73, aricept (donepezil), ASP-1702/CTS-21166, ASP-2905, ASP0777, AV-965, avagacestat (BMS-708163), AVN-101, AVN-322, AVN-397, AZD0328, AZD1446/TC-6683, AZD3839, AZD5213, BAN2401, bapineuzumab, begacestat (PF-05212362), BIIB037, bisnorcymserine, BLU-8499, BMS-241027, BMS-933043, CAD-106, CERE-110 (AAV-NGF), cerlapirdine (PF-05212365/SAM-531), chantix (varenicline tartrate), CHF5074, crenezumab (RG7412), davunetide (AL-108), dimebon (latrepirdine), DM-99, DSP-8658, DWP09031, E2012, E2212, E2609, ELND005 (scyllo-inositol), ELND006, etazolate (EHT 0202), EVP-0962, EVP-6124, Exebryl-1, exelon (rivastigmine), gammagard (immunoglobulin), gantenerumab (RG1450), GSK-239512, GSK1034702, GSK933766, HPP854, INM-176, irdabisant (CEP-26401), ispronicline (AZD3480), K-828-AB, ladostigil tartrate (TV-3326), LMTX (TRx0237), LNK-754, Lu AE58054, LY2886721, masitinib (AB-1010), MCD-386, methanesulfonyl fluoride (SNX-001), mibampator (LY451395), mitoglitazone (MSDC-0160), MK-0249, MK-8931, namenda (memantine), namisol (dronabinol), NIC5-15, nilvadipine (ARC-029), NsG0202, nuedexta (dextromethorphan/quinidine), nypta (tideglusib), octagam (immunoglobulin), ORM-12741, PBT2, PF-03654746, PF-044467943, PF-04995274, PF-05212377/SAM-760, PF-05297909, ponezumab (PF-04360365), posiphen (phenserine), pozanicline tartrate (ABT-089), PQ912, razadyne (galantamine), rember, RG1577/EVT 302, RG3487/MEM-3454, RG7129, rilapladib (SB-659032), RPh-201, RQ-9, RVX-208, S 38093, S 47445/CX1632, SAR110894, SAR228810, SB-742457, SB-742457, semagacestat (LY450139), SEP-227900, sGC-106, solanezumab (LY2062430), SRA-444, ST101/ZSET-1446, SUVN-502, SYN120, T-817MA, TAK-065, TAK-357, TC-5619, TTP-488/PF-04494700, TTP4000, UB-311, V950, velusetrag (TD-5108), VI-1121, VP4896 (leuprolide acetate), abilify (aripiprazole), ABI009/01, ABT-436, ADX-71149, ALKS 5461 (buprenorphine/samidorphan), ARA-290, AVN-628, AZD-2066, AZD-2327, AZD-6765, AZD-7268, BC-22, BCI-1038, BCI-1206, BCI-1283, BCI-224 (sabcomeline), BCI-540 (coluracetam), BCI-632, BCI-838, BCI-952, BMS-820836, BMS-866949, BNC-210, brexpiprazole (OPC-34712), C4X-600 series, cariprazine (RGH-188), CAT-320, CLR-201, CLR-3001, CLR-316, CP-601,927, cymbalta (duloxetine), DSP-1053, E-2508, EB-1010 (amitifadine), edivoxetine (LY2216684), escitalopram, esketamine, EVT 103, EVT-101, filorexant (MK-6096), GLYX-13, GSK-163090, GSK-561679 (verucerfont), GSK1360707, GSK206136, GSK424887, GSK586529, GSK856553 (losmapimod), GW-823296 (orvepitant), GW876008 (emicerfont), HT-2157, JZAD-IV-22, ketamine, latuda (lurasidone), levomilnacipran (F2695), LY2940094, MCP-203, MK-8800, MPP-22 (progestomat), NLF-10808, NPT-500, NSD-788, NSI-189, Org-24448 (farampator), Org-26576, paroxetine/clonazepam, PEL-576, PNB-01 (pipamperone/citalopram), pristiq (desvenlafaxine), PSN-0041, RG-1578, RG-7090, RG7166, RG7351, SA-4503 (cutamesine), SEP-225289, SEP-227162, SEP-228432, serdaxin (clavulanic acid), seroquel XR (quetiapine extended release), SKL-DEP, SPN-809, SRX-246, SRX-251, SSR125543, SSR411298, SUVN-90121, SUVN-911, tasimelteon (VEC-162), TC-5214, tedatioxetine (Lu AA24530), TGBA01A, TGFK07AD (gepirone ER), tianeptine, triRima (CX157), UR8880 (cimicoxib), valdoxan (agomelatine), viibryd (vilazodone), viotra, vortioxetine (Lu AA21004), vyvanse (lisdexamfetamine), WAY-253752, WP-206, WS-50030, circadin (melatonin), silenor (doxepin), tasimelteon (VEC-162), SKP-1041 (zaleplon), MK-6096, GSK649868, Neu-P11, aripiprazole Lauroxil (ALKS 9070), bitopertin (RG1678), BL-1020, cariprazine, davunetide, EVP-6124, fanapt (iloperidone), geodon (ziprasidone), GSK-239512, invega (paliperidone), latuda (lurasidone), LY2140023, OPC-34712, (brexpiprazole), pimavanserin tartrate, risperdal (risperidone), saphris (asenapine), seroquel (quetiapine), TG-5619, vyvanse (lisdexamfetamine), zicronapine (Lu 31-130), zyprexa (olanzapine), topiramate, diclofenac, botox (onabotulinum toxin A), actelion-2, RG-3477, albu-interferon-beta, alpha-cobratoxin RPI-78M, alpha-fetoprotein (MM-093), ampyra (fampridine), Anti-CD4 mAb, arbaclofen placarbil (XP-19986), ASP-4058, ATL-1102, ATX-MS-1467, BAF-312, baminercept, beta-hCG, erythropoietin, BG-12 (dimethyl fumarate), BHT-3009, BIIB-017 (PEG-IFN-beta1a), BIIB-033 LINGO-1 antibody, CCX-140, CDP323, cladribine, CS-0777, cyclophosphamide, DA-3051, daclizumab, dirucotide (MBP8298), dronabinol, ELND-002, firategrast (SB-683699), GAI-122, GBR-500, gilenya (fingolimod), GSK-1223249, GSK-2018682, idebenone (SNT-MC17), IFN-beta1a, IFN-beta1b, IMO-3100, glatiramer acetate, interferon gamma, interleukin 23 antibody LY-2525623, IPX-056, laquinimod, LAS-186323, lemtrada (alemtuzumab), LY-2127399, masitinib (AB-1010), MCT-125 metalloenzyme inhibitors V85546, methylhydrogene fumarate prodrug, MIS-416, cladribine, MSC1-C15, MT-204, mTOR kinase inhibitors NNZ-4921, ocrelizumab (RG-1594), ofatumumab (GSK-1841157), ONO-4641 PAP-1, PEG-interferon beta, perampanel (E2007), PG-102, PI-2301 plovamer, pixantrone, PKC theta inhibitors, pleneva (BGC-20-0134), PLX-MS, PPAR-delta agonist, PS-031291, PV-267, revamilast GRC-4039, , SB-618, secukinumab (AIN-457), ShK-186, sphingosine-1-phosphate agonist, sulfatide, teriflunomide, thymosin β4 based peptide, tranilast MK-341, trimesta (Estriol), VAP1 antagonist, VSN-16, aciclovir, VX-15, VX-5, XToII (chaperonin-10), ZAP-70 inhibitors, ABT-652, ADL5747, ADL5859, afimoxifene, ARRY-797, asimadoline, ASP-6973, axomadol, betamethasone, methocarbamol, indomethacin, ibuprofen, CG100649, CNSB004, CNV2197944, COV795, danazol, dexmedetomidine, E52862, E58425, fasitibant chloride, FX-005, FX006, GRT-6005, GRT-6006, GRT6011, GRT6012, INT-0028, IP-045, JNJ-39439335, JNJ42160443 / AMG403 (fulranumab), LY-3023703, LY-3031207, MCP201, MEDI-5117, nalbuphine, NCE1, NCE2, NCE3, NKTR181, NKTR181(1), NP-2, Diamyd (NP2 enkephalin), NT-11624, PF-04531083, PF-05089771 , PHN131, PPC5650, RQ-00000007, SAR 164877, SAR113945, SAR292833 (GRC15300), SYN 1002, tanezumab, tectin (tetrodotoxin), tilidine hydrochloride, TPH023, TT-063 (S-flurbiprofen), V158866, Xen-2174, XEN402, Z-160, Z-944, ZP002 (8) ADL5859, Anavex1007, Anavex1066, Anavex1519, ARRY797, CR845, Cyt1010, END-3010, END-3020, MK49, NDL-101, Neo1509, NGX-6052, NGX5752, NGX9674 (prodrug of acetaminophen), OX27, OX51, PRF-108 (ropivacaine), RQ00000008, RQ00317076, THA901, THA902, THA903, TPW-196, , Xen2174,ABT102, AFC-5261, ASP8477, AV-1013, AV101, CNSB002, CNSB007, CNV2197944, CXB-909, Cymbalta (duloxetine), CYT-1010, Debio0827 (PL37), DWP05195, EHT/AGN 0001, EMA401, EVT-101, GRC 17536, GRC-10693 (tedalinab), GRC10693, GRC15300 (SAR292833), HF0299, IPI940, KHK6188, Nav 1.3 Antagonist, Neublastin, NGX1998 (capsaicin), NGX426 (tezampanel prodrug), NRX, NSG33, NXN462, PHE377, PRS-639,058, ralfinamide (NW-1029), SAR114137, SD118, SD254, SEP-228432, SKL-NP, SYN1002, TD-9855, USL260, XEN403, ABT-652, ADL5747, ADL5859, afimoxifene, ARRY-797, asimadoline, ASP-6973, axomadol, betamethasone, methocarbamol, indomethacin, ibuprofen, CG100649, CNSB004, CNV2197944, COV795, danazol, dexmedetomidine, E52862, E58425, fasitibant chloride, FX-005, FX006, GRT-6005, GRT-6006, GRT6011, GRT6012, INT-0028, IP-045 , JNJ-39439335, JNJ42160443 / AMG403 (fulranumab), LY-3023703, LY-3031207, MCP201, MEDI-5117, nalbuphine, NCE1, NCE2, NCE3, NKTR181, NKTR181(1), NP-2, Diamyd (NP2 enkephalin), NT-11624, PF-04531083, PF-05089771 , PHN131, PPC5650, RQ-00000007, SAR 164877, SAR113945, SAR292833 (GRC15300), SYN 1002, tanezumab, tectin (tetrodotoxin), tilidine hydrochloride, TPH023, TT-063 (S-flurbiprofen), V158866, Xen-2174, XEN402, Z-160, Z-944, ZP002 (8) ADL5859, Anavex1007, Anavex1066, Anavex1519, ARRY797, CR845, Cyt1010, END-3010, END-3020, MK49, NDL-101, Neo1509, NGX-6052, NGX5752, NGX9674 (prodrug of acetaminophen), OX27, OX51, PRF-108 (ropivacaine), RQ00000008, RQ00317076, THA901, THA902, THA903, TPW-196, , Xen2174,ABT102, AFC-5261, ASP8477, AV-1013, AV101, CNSB002, CNSB007, CNV2197944, CXB-909, Cymbalta (duloxetine), CYT-1010, Debio0827 (PL37), DWP05195, EHT/AGN 0001, EMA401, EVT-101, GRC 17536, GRC-10693 (tedalinab), GRC10693, GRC15300 (SAR292833), HF0299, IPI940, KHK6188, Nav 1.3 Antagonist, Neublastin, NGX1998 (capsaicin), NGX426 (tezampanel prodrug), NRX, NSG33, NXN462, PHE377, PRS-639,058, ralfinamide (NW-1029), SAR114137, SD118, SD254, SEP-228432, SKL-NP, SYN1002, TD-9855, USL260, XEN403, or any combination thereof.

In a preferred embodiment, the VLP according to the invention is loaded with a drug selected from the group consisting of 4-aminopyridine, acamprosate, ademetionine, agomelatine, almorexant, alprazolam, amisulpride, amitriptyline, amitriptylinoxide, amphetamine, anatibant, apomorphine, aripiprazole, armodafinil, asenapine, asenapine, atomoxetine, AZD2066, AZD2423, AZD3839, bapineuzumab, benserazide, betahistine, bifeprunox, blonanserin, bromazepam, brotizolam, buprenorphine, bupropion, buspirone, carbamazepine, carbidopa, Carpipramine, CDP323, celecoxib, citalopram, cladribine, clobazam, clomipramine, clonazepam, clonidine HCL, clozapine, dalfampridine, desflurane, desipramine, desvenlafaxine, dexketoprofen trometamol, dextromethorphan, quinidine sulfate, diazepam, dihydroergotamine mesylate, dimethyl fumarate, sodium valproate, valproic acid, donepezil, dosulepin, doxepin, droxidopa, duloxetine, edaravone, eletriptan, entacapone, escitalopram, eslicarbazepine acetate, eszopiclone, etizolam, ezogabine, fentanyl, fingolimod, fluoxetine, flupirtine, fluvoxamine, fluvoxamine, fosphenytoin, fospropofol, frovatriptan, gabapentin, galantamine, glatiramer acetate, glycopyrrolate, guanfacine, ghrelin, hyaluronic acid, hydrocodone, hydromorphone, hydroxyzine, ibandronic acid, iloperidone, imipramine, indometasin, interferon beta-1a, iproniazid, isocarboxazid, istradefylline, ketorolac tromethamine, lacosamide, lamotrigine, levetiracetam, levodopa, lisdexamfetamine, lofepramine, lormetazepam, Lu02-750, LuAA24493, LuAA34893, LuAA39959, LuAE04621, lurasidone, maprotiline, MEDI-578, memantine, methylphenidate, mianserin, milnacipran, mirtazapine, mitoxantrone, moclobemide, modafinil, morphine, nalmefene, naltrexone, naproxen, naratriptan, natalizumab, nefazodone, nortriptyline, novantrone, olesoxime, olanzapine, oxcarbazepine, oxitriptan, oxycodone, paliperidone, paracetamol, pardoprunox, paroxetine, perampanel, pergolide, perospirone, phenelzine, phenytoin, piracetam, piribedil, pozanicline tartrate, pramipexole, pregabalin, propofol, protriptyline, quazepam, quetiapine, ramelteon, rasagiline, reboxetine, retigabine, rilmazafone, riluzole, risperidone, rivastigmine, rizatriptan, rocuronium bromide, ropinirole, rotigotine, rufinamide, selegiline, sertindole, sertraline, setiptiline, sevoflurane, SLV334, sodium oxybate, sugammadex, sulbutiamine, sulpiride, sumatriptan, tafamidis meglumine, taltireline, tandospirone, tapentadol, TC-5214, TC-5619, telcagepant, teriflunomide, tetrabenazine, tianeptine, tiapride, tissue plasminogen inhibitor (tPA), tizanidine, topiramate, tramadol, tranylcypromine, trazodone, trimipramine, tryptophan, varenicline, venlafaxine, vigabatrin, viloxazine, ziprasidone, zolmitriptan, zolpidem, zonisamide, a dihydropyridin calcium channel blocker such as amlodipine, aranidipine, azelnidipine, barnidipine, benidipine, cilnidipine, clevidipine, cronidipine, darodipine, dexniguldipine, efonidipine, elnadipine, elgodipine, felodipine, flordipine, furnidipine, iganidipine, isradipine, lacidipine, lemildipine, lercanidipine, manidipine, mesuldipine, nicardipine, nifedipine, niguldipine, nimodipine, niludipine, nilvadipine, nisoldipine, nitrendipine, olradipine, oxodipine, palonidipine, pranidipine, sagandipine, sornidipine, teludipine, tiamdipine, trombodipine, watanidipine, ABT354, ABT363, ABT436, ABT560, ADX48621, amantadine, AMG 579, AMG 747, Anti Lingo, ARX 424, ASP0777, ASP2905, ATI355, ATX-MS-1467, AZD1446, beta Secretase Inhibitor, BIIB037 (BART), BMS241027, CS-0777, CX1010, elpetrigine, FK949E, GRT-6006, GSK1223249, GSK356278, LuAF11167, MK-4409, MK-7288, MK-8931, naluzotan, Neublastin, ordopidine, PI-2301, R1662, R7129, R7314, SAR127963, SAR228810, SB623, SEP-225441, SEP-228432, SPN802, SUN13837, T2007, TAK-065, TAK-937, TD-8954, TD-9855, UCB0942, V950, PF-04427429, PF-04531083, PF-04958242, PF-05180999, PF-05236812, ABT110, ABT126, ABT288, ABT384, ABT639, ABT652, ABT894, ACC-001, AFQ056, AGN209323, ALKS 33, AZD5213, AZD6765, BAF312, BGG492, BI 44370 TA, BMS708163, BMS820836, BMS927711, BMS954561, bupivacaine, CAD106, caprylic triglyceride, creatine PD-02, CX-717, Davunetide/AL-108, Debio-9902, DS-5565, EHT-0202, ELND-005, fipamezole (BVF025, JP1730), Firategrast, fulranumab, Ganaxolone, GRT-6005, GSK249320, GSK561679, GSK649868, GSK742457, GSK933776, HF0220, HT-0712, Huperzine A, ICA-105665, ispronicline, JNJ-26489112, Lu AA24530, LuAE58054, LY2062430, MCC-257, MEM-3454, methylthioninium chloride, MK-0249, MK-3697, MK-6096, naldemedine, NIC002, NIC5-15, NP002, ONO-4641, OPC-249, PBT2, PF-02545920, PF-03049423, PF-03654746, PF-04360365, PF-04494700 (TTP488), PF-05089771, PF-05236806, plasmid encoding aromatic L-amino acid decarboxylase, PRX-03140, R1578, R7090, R7412, radiprodil, S-33138, S-90098, SAM-531, SAR110894, SAR292833, SB-742457, SEP-225289, SEP-227162, simvastatin, SSR-180711, TA-5538, TAK-428, talampanel, tedatioxetine, TV-1102, VX-765, YKP3089, ABT957, ALKS 36, camor, MRZ-8676, and rHIgM22 or any combination thereof.

In an even more preferred embodiment the VLP according to the invention is loaded with a drug selected from the group consisting of 4-aminopyridine, acamprosate, ademetionine, agomelatine, almorexant, alprazolam, amisulpride, amitriptyline, amitriptylinoxide, amphetamine, anatibant, apomorphine, aripiprazole, armodafinil, asenapine, asenapine, atomoxetine, AZD2066, AZD2423, AZD3839, bapineuzumab, benserazide, betahistine, bifeprunox, blonanserin, bromazepam, brotizolam, buprenorphine, bupropion, buspirone, carbamazepine, carbidopa, Carpipramine, CDP323, celecoxib, citalopram, cladribine, clobazam, clomipramine, clonazepam, clonidine HCL, clozapine, dalfampridine, desflurane, desipramine, desvenlafaxine, dexketoprofen trometamol, dextromethorphan, quinidine sulfate, diazepam, dihydroergotamine mesylate, dimethyl fumarate, sodium valproate, valproic acid, donepezil, dosulepin, doxepin, droxidopa, duloxetine, edaravone, eletriptan, entacapone, escitalopram, eslicarbazepine acetate, eszopiclone, etizolam, ezogabine, fentanyl, fingolimod, fluoxetine, flupirtine, fluvoxamine, fluvoxamine, fosphenytoin, fospropofol, frovatriptan, gabapentin, galantamine, glatiramer acetate, glycopyrrolate, guanfacine, ghrelin, hyaluronic acid, hydrocodone, hydromorphone, hydroxyzine, ibandronic acid, iloperidone, imipramine, indometasin, interferon beta-1a, iproniazid, isocarboxazid, istradefylline, ketorolac tromethamine, lacosamide, lamotrigine, levetiracetam, levodopa, lisdexamfetamine, lofepramine, lormetazepam, Lu02-750, LuAA24493, LuAA34893, LuAA39959, LuAE04621, lurasidone, maprotiline, MEDI-578, memantine, methylphenidate, mianserin, milnacipran, mirtazapine, mitoxantrone, moclobemide, modafinil, morphine, nalmefene, naltrexone, naproxen, naratriptan, natalizumab, nefazodone, nortriptyline, novantrone, olesoxime, olanzapine, oxcarbazepine, oxitriptan, oxycodone, paliperidone, paracetamol, pardoprunox, paroxetine, perampanel, pergolide, perospirone, phenelzine, phenytoin, piracetam, piribedil, pozanicline tartrate, pramipexole, pregabalin, propofol, protriptyline, quazepam, quetiapine, ramelteon, rasagiline, reboxetine, retigabine, rilmazafone, riluzole, risperidone, rivastigmine, rizatriptan, rocuronium bromide, ropinirole, rotigotine, rufinamide, selegiline, sertindole, sertraline, setiptiline, sevoflurane, SLV334, sodium oxybate, sugammadex, sulbutiamine, sulpiride, sumatriptan, tafamidis meglumine, taltireline, tandospirone, tapentadol, TC-5214, TC-5619, telcagepant, teriflunomide, tetrabenazine, tianeptine, tiapride, tissue plasminogen inhibitor (tPA), tizanidine, topiramate, tramadol, tranylcypromine, trazodone, trimipramine, tryptophan, varenicline, venlafaxine, vigabatrin, viloxazine, ziprasidone, zolmitriptan, zolpidem, and zonisamide, a dihydropyridin calcium channel blocker such as amlodipine, aranidipine, azelnidipine, barnidipine, benidipine, cilnidipine, clevidipine, cronidipine, darodipine, dexniguldipine, efonidipine, elnadipine, elgodipine, felodipine, flordipine, furnidipine, iganidipine, isradipine, lacidipine, lemildipine, lercanidipine, manidipine, mesuldipine, nicardipine, nifedipine, niguldipine, nimodipine, niludipine, nilvadipine, nisoldipine, nitrendipine, olradipine, oxodipine, palonidipine, pranidipine, sagandipine, sornidipine, teludipine, tiamdipine, trombodipine, watanidipine, or any combination thereof.

According to the invention the VLP for use as a drug delivery system can be used for the treatment or diagnosis of neurological disorders.

According to the invention the term "neurological disorder" refers to any disorder of the body's nervous system, preferably of the central nervous system. It encompasses neurodegenerative, psychotic or neurovascular disorders.

In an embodiment of the invention the neurological disorder is selected from the group consisting of stroke, alcohol addiction, Alzheimer Disease, anxiety, arthritis, asthenia, attention deficit hyperactivity disorder, bipolar disorder, cancer pain, cerebral ischemia, cerebral neuroprotectant, cervical dystonia, Chorea associated with Huntington's disease, chronic pain, chronic severe pain, cognitive disorder, cortical myoclonus, degenerative Nerve Diseases, depression, diabetic neuropathic pain, diabetic neuropathy, emotional lability, epilepsy, excessive sleepiness associated with narcolepsy, fibromyalgia, Fragile X syndrome, Friedreich's ataxia, insomnia, Lennox Gastaut syndrome, major depressive and anxiety disorders, manic episodes associated with bipolar disorder, memory impairment, migraine, mild cognitive impairment, moderate to severe pain, motor neuron disease, multiple sclerosis, musculoskeletal pain, narcolepsy, neuralgia, neuropathic pain, nicotine dependence, obsessive compulsive disorder, opioid-induced adverse effect, opioid-induced constipation, osteoarthritis pain, overactive bladder, pain, Parkinson's disease, pediatric drooling, peripheral diabetic neuropathy, post-operative pain, postherpetic neuralgia, premenstrual dysphoric disorder, psychosis, refractory complex partial seizures, restless leg syndrome (RLS), schizophrenia, seizure, severe chronic pain, sleep disorder, smoking cessation, spasticity, spinal cord injury, stroke, transthyretin familial amyloid polyneuropathy, traumatic brain injury, vertigo, cachexia, amyotrophic lateral sclerosis, spinocerebellar ataxia type I, extrapyramidal and movement disorders, transient ischemic attack (TIA), Progressive multifocal leukoencephalopathy (PML), HIV-infection, dementia, such as Alzheimer's disease, vascular dementia, frontotemporal dementia, semantic dementia and dementia with Lewy bodies, and preferably selected from the group consisting of Alzheimer's disease, Parkinson's disease and multiple sclerosis.

In a preferred embodiment of the invention, the neurological disorder to be treated or diagnosed with the VLP of the invention is Alzheimer's disease.

In another preferred embodiment of the invention, the neurological disorder to be treated or diagnosed with the VLP of the invention is Parkinson's disease.

In a further on preferred embodiment of the invention, the neurological disorder to be treated or diagnosed with the VLP of the invention is multiple sclerosis.

In a further embodiment of the invention, the VLP when used for diagnosis or treatment of multiple sclerosis is loaded with a drug from the group consisting of ARX 424, dalfampridine, mitoxantrone, natalizumab, novantrone, TV-1102, , actelion-2, RG-3477, albu-interferon-beta, alpha-cobratoxin RPI-78M, alpha-fetoprotein (MM-093), ampyra (fampridine), Anti-CD4 mAb, arbaclofen placarbil (XP-19986), ASP-4058, ATL-1102, ATX-MS-1467, BAF-312, baminercept, beta-hCG, erythropoietin, BG-12 (dimethyl fumarate), BHT-3009, BIIB-017 (PEG-IFN-beta1a), BIIB-033 LINGO-1 antibody, CCX-140, CDP323, cladribine, CS-0777, cyclophosphamide, DA-3051, daclizumab, dirucotide (MBP8298), dronabinol, ELND-002, firategrast (SB-683699), GAI-122, GBR-500, gilenya (fingolimod), GSK-1223249, GSK-2018682, idebenone (SNT-MC17), IFN-beta1a, IFN-beta1b, IMO-3100, glatiramer acetate, interferon gamma, interleukin 23 antibody LY-2525623, IPX-056, laquinimod, LAS-186323, lemtrada (alemtuzumab), LY-2127399, masitinib (AB-1010), MCT-125 metalloenzyme inhibitors V85546, methylhydrogene fumarate prodrug, MIS-416, cladribine, MSC1-C15, MT-204, mTOR kinase inhibitors NNZ-4921, ocrelizumab (RG-1594), ofatumumab (GSK-1841157), ONO-4641 PAP-1, PEG-interferon beta, perampanel (E2007), PG-102, PI-2301 plovamer, pixantrone, PKC theta inhibitors, pleneva (BGC-20-0134), PLX-MS, PPAR-delta agonist, PS-031291, PV-267, revamilast GRC-4039, , SB-618, secukinumab (AIN-457), ShK-186, sphingosine-1-phosphate agonist, sulfatide, teriflunomide, thymosin β4 based peptide, tranilast MK-341, trimesta (Estriol), VAP1 antagonist, VSN-16, aciclovir, VX-15, VX-5, XToII (chaperonin-10), ZAP-70 inhibitors or any combination thereof.

In another embodiment of the invention, the VLP when used for diagnosis or treatment of Alzheimer's disease is loaded with a drug from the group consisting of ABT363, ABT957, BMS708163, CX-717, Debio-9902, dimebolin hydrochloride, donepezil, HF0220, huperzine A, latrepirdine, LY2062430, LY450139, methylthioninium chloride, PF-05236806, PF-05236812, PRX-03140, RG7412, rosiglitazone, SAM-531, simvastatin, SSR-180711, TD-8954, V950, caprylic triglyceride, HT-0712, RG1662, GSK742457, davunetide/AL-108" AAB-003, AAD-2004, ABT-126, ABT-288, ABT-384, ABT-560, ACC-001 (vanutide cridificar), ACI-24, ACI-91 (pirenzepine), acrescent (memantine/donepezil), actos (pioglitazone), AD-01, AD02, AD03, albutein + flebogamma (albumin/immunoglobulin), ANAVEX-2- 73, aricept (donepezil), ASP-1702/CTS-21166, ASP-2905, ASP0777, AV-965, avagacestat (BMS-708163), AVN-101, AVN-322, AVN-397, AZD0328, AZD1446/TC-6683, AZD3839, AZD5213, BAN2401, bapineuzumab, begacestat (PF-05212362), BIIB037, bisnorcymserine, BLU-8499, BMS-241027, BMS-933043, CAD-106, CERE-110 (AAV-NGF), cerlapirdine (PF-05212365/SAM-531), chantix (varenicline tartrate), CHF5074, crenezumab (RG7412), davunetide (AL-108), dimebon (latrepirdine), DM-99, DSP-8658, DWP09031, E2012, E2212, E2609, ELND005 (scyllo-inositol), ELND006, etazolate (EHT 0202), EVP-0962, EVP-6124, Exebryl-1, exelon (rivastigmine), gammagard (immunoglobulin), gantenerumab (RG1450), GSK-239512, GSK1034702, GSK933766, HPP854, INM-176, irdabisant (CEP-26401), ispronicline (AZD3480), K-828-AB, ladostigil tartrate (TV-3326), LMTX (TRx0237), LNK-754, Lu AE58054, LY2886721, masitinib (AB-1010), MCD-386, methanesulfonyl fluoride (SNX-001), mibampator (LY451395), mitoglitazone (MSDC-0160), MK-0249, MK-8931, namenda (memantine), namisol (dronabinol), NIC5-15, nilvadipine (ARC-029), NsG0202, nuedexta (dextromethorphan/quinidine), nypta (tideglusib), octagam (immunoglobulin), ORM-12741, PBT2, PF-03654746, PF-044467943, PF-04995274, PF-05212377/SAM-760, PF-05297909, ponezumab (PF-04360365), posiphen (phenserine), pozanicline tartrate (ABT-089), PQ912, razadyne (galantamine), rember, RG1577/EVT 302, RG3487/MEM-3454, RG7129, rilapladib (SB-659032), RPh-201, RQ-9, RVX-208, S 38093, S 47445/CX1632, SAR110894, SAR228810, SB-742457, SB-742457, semagacestat (LY450139), SEP-227900, sGC-106, solanezumab (LY2062430), SRA-444, ST101/ZSET-1446, SUVN-502, SYN120, T-817MA, TAK-065, TAK-357, TC-5619, TTP-488/PF-04494700, TTP4000, UB-311, V950, velusetrag (TD-5108), VI-1121 and VP4896 (leuprolide acetate) or any combination thereof.

In another embodiment of the invention, the VLP when used for diagnosis or treatment of Parkinson's disease is loaded with a drug from the group consisting of ADX48621, AFQ056, amantadine, apomorphine, camor, carbidopa, creatine PD-02, fipamezole (BVF025, JP1730), levodopa, MRZ-8676, naluzotan, NP002, ordopidine, safinamide, pardoprunox, a plasmid encoding aromatic L-amino acid decarboxylase, piribedil, AZD3839, benserazide, droxidopa, entacapone, GSK587124, istradefylline, Lu02-750, LuAE04621, pergolide, preladenant, rasagiline, rotigotine, pramipexole, ABT-652, ADL5747, ADL5859, afimoxifene, ARRY-797, asimadoline, ASP-6973, axomadol, betamethasone, methocarbamol, indomethacin, ibuprofen, CG100649, CNSB004, CNV2197944, COV795, danazol, dexmedetomidine, E52862, E58425, fasitibant chloride, FX-005, FX006, GRT-6005, GRT-6006, GRT6011, GRT6012, INT-0028, IP-045 , JNJ-39439335, JNJ42160443 / AMG403 (fulranumab), LY-3023703, LY-3031207, MCP201, MEDI-5117, nalbuphine, NCE1, NCE2, NCE3, NKTR181, NKTR181(1), NP-2, Diamyd (NP2 enkephalin), NT-11624, PF-04531083, PF-05089771 , PHN131, PPC5650, RQ-00000007, SAR 164877, SAR113945, SAR292833 (GRC15300), SYN 1002, tanezumab, tectin (tetrodotoxin), tilidine hydrochloride, TPH023, TT-063 (S-flurbiprofen), V158866, Xen-2174, XEN402, Z-160, Z-944, ZP002 (8) ADL5859, Anavex1007, Anavex1066, Anavex1519, ARRY797, CR845, Cyt1010, END-3010, END-3020, MK49, NDL-101, Neo1509, NGX-6052, NGX5752, NGX9674 (prodrug of acetaminophen), OX27, OX51, PRF-108 (ropivacaine), RQ00000008, RQ00317076, THA901, THA902, THA903, TPW-196, , Xen2174,ABT102, AFC-5261, ASP8477, AV-1013, AV101, CNSB002, CNSB007, CNV2197944, CXB-909, Cymbalta (duloxetine), CYT-1010, Debio0827 (PL37), DWP05195, EHT/AGN 0001, EMA401, EVT-101, GRC 17536, GRC-10693 (tedalinab), GRC10693, GRC15300 (SAR292833), HF0299, IPI940, KHK6188, Nav 1.3 Antagonist, Neublastin, NGX1998 (capsaicin), NGX426 (tezampanel prodrug), NRX, NSG33, NXN462, PHE377, PRS-639,058, ralfinamide (NW-1029), SAR114137, SD118, SD254, SEP-228432, SKL-NP, SYN1002, TD-9855, USL260, XEN403 and ropinirole or any combination thereof.

In a further embodiment of the invention, the VLP when used for diagnosis or treatment of stroke is loaded with a drug selected from the group consisting of tissue-plaminogen activator, desmoteplase, GSK249320, edaravone, SB623, PF-03049423 and TAK-937 or any combination thereof.

In another embodiment of the invention, the VLP when used for diagnosis or treatment of attention deficit hyperactivity disorder (ADHD) is loaded with a drug selected from the group consisting of atomoxetine, amphetamine, clonidine HCl, lisdexamfetamine, pozanicline tartrateabilify (aripiprazole), ABT089 (pozanicline), ABT894 (sofnicline), aricept (donepezil), AZ3480/TC1734 (ispronicline), AZD1446, AZD5213, bavisant/JNJ31001074, guanfacine, ampakine, droxidopa (L-dihydroxyphenyl-serine), DU28853/PGI256 (eltoprazine), KP106 (prodrug of D-amphetamine), lobeline, LY2216684 (edivoxetine), MG01CI (metadoxine), MK0249, ND0801, NERI IV, methylphenidate, OPC-34712, PD9475 (betahistine), PF3654746, SPN811, SPN812, TC-5619, and zalvari/SPN810 (molindone) or any combination thereof.

In another embodiment of the invention, the VLP when used for diagnosis or treatment of drug dependency or addiction is loaded with a drug selected from the group consisting of ALKS 33, naltrexone, nalmefene, varenicline, bupropion, NIC002 and acamprosate or any combination thereof.

In a further embodiment of the invention, the VLP when used for diagnosis or treatment of amyotrophic lateral sclerosis is loaded with a drug selected from the group consisting of dexpramipexole, GSK1223249, mecobalamin, olesoxime, riluzole, ALS02 (creatine monohydrate), E0302 (mecabalamin), CK-2017357 (tirasemtiv), mitogard (cyclosporine), NP001, AEN100 (zinc acetate), RPI-MN, sNN0029, AAD2004, NS1566, KP100IT, MoNuDin, Cogane, GVT (monosodium luminol), AS101, CERE135 (AAV-AGF-1), Nrp2945, DP-460, GM604, noscapine, LB-200, Hsp90 inhibitor, NT-K0-003, VAR-10100, IPL-344, CNS-102 and talampanel or any combination thereof.

In another embodiment of the invention, the VLP when used for diagnosis or treatment of anxiety disorder is loaded with a drug selected from the group consisting of hydroxyzine, tandospirone, buspirone, divalproex ER, bromazepam and SEP-225441 or any combination thereof.

In one embodiment of the invention, the VLP when used for diagnosis or treatment of autism is loaded with a drug selected from a group consisting of amantadine, ampakine (CX-516), arbaclofen (STX-209), aripiprazole, atomoxetine (Strattera), carbetocin, citalopram, D-cycloserine, D-serine, fluoxetine (NPL-2008), guanfacine, lamotrigine, lenalidomide (Revlimid), memantine hydrochloride (Namenda), methylphenidate, N-acetylcysteine, oxytocin, paliperidone, riluzole, risperidone, sapropterin (Kuvan), STX107, synthetic human secretin (RG1068), synthetic porcine secretin and ChiRhoClin, or any combination thereof.

In a further embodiment of the invention, the VLP when used for diagnosis or treatment of major depressive disorder is loaded with a drug selected from the group consisting of ABT436, ademetionine, agomelatine, amitriptyline, amitriptylinoxide, carpipramine, citalopram, clomipramine, desipramine, desvenlafaxine, dosulepin, doxepin, etizolam, fluoxetine, fluvoxamine, imipramine, iproniazid, isocarboxazid, JNJ-26489112, lofepramine, LuAA34893, LY-2216684 (NERI IV), maprotiline, mianserin, milnacipran, mirtazapine, moclobemide, nefazodone, nortriptyline, olanzapine, oxitriptan, paroxetine, phenelzine, protriptyline, RG1578, RG7090, reboxetine, selegiline, sertraline, setiptiline, tranylcypromine, trazodone, trazodone, trimipramine, tryptophan, venlafaxine, viloxazine, vortioxetine, HT-0712, R1662, TC-5619, amisulpride, FK949E, S-90098, , abilify (aripiprazole), ABI009/01, ABT-436, ADX-71149, ALKS 5461 (buprenorphine/samidorphan), ARA-290, AVN-628, AZD-2066, AZD-2327, AZD-6765, AZD-7268, BC-22, BCI-1038, BCI-1206, BCI-1283, BCI-224 (sabcomeline), BCI-540 (coluracetam), BCI-632, BCI-838, BCI-952, BMS-820836, BMS-866949, BNC-210, brexpiprazole (OPC-34712), C4X-600 series, cariprazine (RGH-188), CAT-320, CLR-201, CLR-3001, CLR-316, CP-601,927, cymbalta (duloxetine), DSP-1053, E-2508, EB-1010 (amitifadine), edivoxetine (LY2216684), escitalopram, esketamine, EVT 103, EVT-101, filorexant (MK-6096), GLYX-13, GSK-163090, GSK-561679 (verucerfont), GSK1360707, GSK206136, GSK424887, GSK586529, GSK856553 (losmapimod), GW-823296 (orvepitant), GW876008 (emicerfont), HT-2157, JZAD-IV-22, ketamine, latuda (lurasidone), levomilnacipran (F2695), LY2940094, MCP-203, MK-8800, MPP-22 (progestomat), NLF-10808, NPT-500, NSD-788, NSI-189, Org-24448 (farampator), Org-26576, paroxetine/clonazepam, PEL-576, PNB-01 (pipamperone/citalopram), pristiq (desvenlafaxine), PSN-0041, RG-1578, RG-7090, RG7166, RG7351, SA-4503 (cutamesine), SEP-225289, SEP-227162, SEP-228432, serdaxin (clavulanic acid), seroquel XR (quetiapine extended release), SKL-DEP, SPN-809, SRX-246, SRX-251, SSR125543, SSR411298, SUVN-90121, SUVN-911, tasimelteon (VEC-162), TC-5214, tedatioxetine (Lu AA24530), TGBA01A, TGFK07AD (gepirone ER), tianeptine, triRima (CX157), UR8880 (cimicoxib), valdoxan (agomelatine), viibryd (vilazodone), viotra, vortioxetine (Lu AA21004), vyvanse (lisdexamfetamine), WAY-253752, WP-206 and WS-50030 or any combination thereof.

In a further embodiment of the invention, the VLP when used for diagnosis or treatment of pain is loaded with a drug selected from the group consisting of nabiximols, AZD2066, AZD2423, DS-5565, SAR292833, AVP-923, ABT110, AGN209323, BMS954561, gabapentin, pregabalin, SEP-228432, neublastin, MEDI-578, ABT639, ABT652, ALKS 36, buprenorphine, carisbamate, fentanyl, fulranumab, GRT-6005, GRT-6006, hydrocodone, hydromorphone, ketorolac tromethamine, MK-4409, morphine, OPC-249, oxycodone, paracetamol, radiprodil, tapentadol, TD-9855, tramadol, 4-aminopyridine, dexketoprofen, trometamol, capsaicin, bupivacaine, PF-04531083, tafamidis meglumine, flupirtin, PF-05089771, ABT-652, ADL5747, ADL5859, afimoxifene, ARRY-797, asimadoline, ASP-6973, axomadol, betamethasone, methocarbamol, indomethacin, ibuprofen, CG100649, CNSB004, CNV2197944, COV795, danazol, dexmedetomidine, E52862, E58425, fasitibant chloride, FX-005, FX006, GRT-6005, GRT-6006, GRT6011, GRT6012, INT-0028, IP-045 , JNJ-39439335, JNJ42160443 / AMG403 (fulranumab), LY-3023703, LY-3031207, MCP201, MEDI-5117, nalbuphine, NCE1, NCE2, NCE3, NKTR181, NKTR181(1), NP-2, Diamyd (NP2 enkephalin), NT-11624, PF-04531083, PF-05089771 , PHN131, PPC5650, RQ-00000007, SAR 164877, SAR113945, SAR292833 (GRC15300), SYN 1002, tanezumab, tectin (tetrodotoxin), tilidine hydrochloride, TPH023, TT-063 (S-flurbiprofen), V158866, Xen-2174, XEN402, Z-160, Z-944, ZP002 (8) ADL5859, Anavex1007, Anavex1066, Anavex1519, ARRY797, CR845, Cyt1010, END-3010, END-3020, MK49, NDL-101, Neo1509, NGX-6052, NGX5752, NGX9674 (prodrug of acetaminophen), OX27, OX51, PRF-108 (ropivacaine), RQ00000008, RQ00317076, THA901, THA902, THA903, TPW-196, , Xen2174,ABT102, AFC-5261, ASP8477, AV-1013, AV101, CNSB002, CNSB007, CNV2197944, CXB-909, Cymbalta (duloxetine), CYT-1010, Debio0827 (PL37), DWP05195, EHT/AGN 0001, EMA401, EVT-101, GRC 17536, GRC-10693 (tedalinab), GRC10693, GRC15300 (SAR292833), HF0299, IPI940, KHK6188, Nav 1.3 Antagonist, Neublastin, NGX1998 (capsaicin), NGX426 (tezampanel prodrug), NRX, NSG33, NXN462, PHE377, PRS-639,058, ralfinamide (NW-1029), SAR114137, SD118, SD254, SEP-228432, SKL-NP, SYN1002, TD-9855, USL260, XEN403 or any combination thereof.

In a further embodiment of the invention, the VLP when used for diagnosis or treatment of Huntington's disease is loaded with the drug tetrabenazine and/or GSK356278.

In a further embodiment of the invention, the VLP when used for diagnosis or treatment of epilepsy or Lennox-Gutault-syndrom is loaded with a drug selected from the group consisting of BGG492, brivaracetam, carbamazepine, CX1010, diazepam, elpetrigine, eslicarbazepine acetate, ezogabine, fosphenytoin, ganaxolone, ICA-105665, lacosamide, lamotrigine, levetiracetam, lyrica (pregabilin), midazolam, oxcarbazepine, perampanel, phenytoin, retigabine, SPN802, T2007, topiramate, UCB0942, VX-765, YKP3089, rufinamide, clobazam, clonazepam vigabatrin and zonisamide or any combination thereof.

In a further embodiment of the invention, the VLP when used for diagnosis or treatment of narcolepsy is loaded with a drug selected from the group consisting of armodafinil, sodium oxybate, MK-7288 or any combination thereof.

In a further embodiment of the invention, the VLP when used for diagnosis or treatment of insomnia is loaded with a drug selected from the group consisting of almorexant, brotizolam, eszopiclone, lormetazepam, MK-3697, MK-6096, quazepam, ramelteon, rilmazafone, suvorexant, modafinil, GSK649868, circadin (melatonin), silenor (doxepin), tasimelteon (VEC-162), SKP-1041 (zaleplon), MK-6096, GSK649868, Neu-P11 and zolpidem or any combination thereof.

In a further embodiment of the invention, the VLP when used for diagnosis or treatment of bipolar disorder is loaded with the drug asenapine.

In a further embodiment of the invention, the VLP when used for diagnosis or treatment of migraine is loaded with a drug selected from the group consisting of BI 44370 TA, BMS927711, dihydroergotamine mesylate, eletriptan, frovatriptan, levadex, naproxen, naratriptan, PF-04427429, rizatriptan, sumatriptan, telcagepant, zolmitriptan, topiramate, diclofenac, botox (onabotulinum toxin A) or any combination thereof.

In a further embodiment of the invention, the VLP when used for diagnosis or treatment of schizophrenia is loaded with a drug selected from the group consisting of ABT354, AMG 747, bifeprunox, blonanserin, clozapine, PF-02545920, PF-04958242, PF-05180999, R1678, S-33138, sertindole, sulpiride, tiapride, zicronapine, LuAA39959, AMG-579, abilify (aripiprazole), ABT-126, loxapine, ADX71149, aripiprazole, aripiprazole Lauroxil (ALKS 9070), bitopertin (RG1678), BL-1020, cariprazine, davunetide, EVP-6124, fanapt (iloperidone), geodon (ziprasidone), GSK-239512, invega (paliperidone), latuda (lurasidone), LY2140023, OPC-34712, (brexpiprazole), pimavanserin tartrate, risperdal (risperidone), saphris (asenapine), seroquel (quetiapine), TG-5619, vyvanse (lisdexamfetamine), zicronapine (Lu 31-130), zyprexa (olanzapine) or any combination thereof.

In a further embodiment of the invention, the VLP when used for diagnosis or treatment of spasticity is loaded with the drug tizanidine.

In a further embodiment of the invention, the VLP when used for diagnosis or treatment of spinal cord injury is loaded with a drug selected from the group consisting of ATI355, rHIgM22, and SUN13837 or any combination thereof.

In a further embodiment of the invention, the VLP when used for diagnosis or treatment of traumatic brain injury is loaded with a drug selected from the group consisting of anatibant, SAR127963 and SLV334 or any combination thereof.

In a further embodiment of the invention, the VLP when used for diagnosis or treatment of vertigo is loaded with the drug betahistine.

In one embodiment of the invention, the VLP when used for diagnosis or treatment of the fragile X syndrome is loaded with the drug mavoglurant.

In another embodiment of the invention, the VLP when used for diagnosis or treatment of the Friedreich's ataxia is loaded with the drug idebenone and/or LuAA24493.

In another embodiment of the invention, the VLP when used for diagnosis or treatment of diabetic neuropathy is loaded with the drug MCC-257 and/or TAK-428.

In another embodiment of the invention, the VLP when used for diagnosis or treatment of cortical myoclonus is loaded with the drug piracetam.

In another embodiment of the invention, the VLP when used for diagnosis or treatment of asthenia is loaded with the drug sulbutiamine.

In another embodiment of the invention, the VLP when used for diagnosis or treatment of cachexia is loaded with the drug ghrelin and preferably human ghrelin.

In a further embodiment of the invention, the VLP when used for diagnosis or treatment of vasospasm associated with subarachnoid hemorrhage is loaded with a drug selected from the group consisting of a dihydropyridin calcium channel blocker such as amlodipine, aranidipine, azelnidipine, barnidipine, benidipine, cilnidipine, clevidipine, cronidipine, darodipine, dexniguldipine, efonidipine, elnadipine, elgodipine, felodipine, flordipine, furnidipine, iganidipine, isradipine, lacidipine, lemildipine, lercanidipine, manidipine, mesuldipine, nicardipine, nifedipine, niguldipine, nimodipine, niludipine, nilvadipine, nisoldipine, nitrendipine, olradipine, oxodipine, palonidipine, pranidipine, sagandipine, sornidipine, teludipine, tiamdipine, trombodipine, watanidipine,

VLP may be produced by providing the desired components, in particular VP1, optionally VP2, optionally VP3 or a mixture thereof and optionally the cargo load, in solution and allowing the components to assembly into the VLP. For example, mixing of the components may be performed under conditions where no or only limited VLP assembly occurs, such as at low Ca²⁺ concentrations and/or reducing conditions, and after addition of all desired components the conditions are changed into those favorable for VLP assembly, such as higher Ca²⁺ concentrations and/or oxidizing conditions. However, VLP production may also occur in vivo. In particular, the components of the VLP may be coexpressed in a host cell and the VLP assemble inside the host cell or upon lysis or disruption of the host cell.

"VP1" or "virus protein 1" according to the present invention refers to a protein which is identical to or is derived from the natural VP1 of the JC virus having the amino acid sequence according to SEQ ID NO: 1. A protein derived from the natural VP1 of the JC virus preferably has an amino acid sequence homology or identity with the amino acid sequence according to SEQ ID NO: 1 of at least 60%, more preferably at least 70%, at least 75%, at least 80%, at least 85%, at least 90%, at least 95%, at least 97%, at least 98% or at least 99% over a sequence of at least 100 contiguous amino acids, preferably at least 150, at least 200, at least 250 or at least 300 contiguous amino acids. Most preferably, the amino acid homology or identity is calculated over the entire length of the natural JCV-VP1. The terms "VP1 derived from the natural VP1 of the JC virus" and "VP1 derived from JC virus" in particular also include VP1 which is identical to the natural VP1 of the JC virus.

The term "VP1" according to the invention also encompasses fractions and derivatives of the natural VP1 which are capable of assembling into VLP. Preferably, said fractions and derivatives of VP1 at least comprise amino acids 32 to 316 of the amino acid sequence according to SEQ ID NO: 1 or a derivative thereof having a homology or identity with the amino acid sequence from amino acid position 32 to 316 of SEQ ID NO: 1 of at least 60%, more preferably at least 70%, at least 75%, at least 80%, at least 85%, at least 90%, at least 95%, at least 97%, at least 98% or at least 99% over a sequence of at least 100 contiguous amino acids, preferably at least 150, at least 200, at least 250 or at least 300 contiguous amino acids, preferably over the entire sequence.

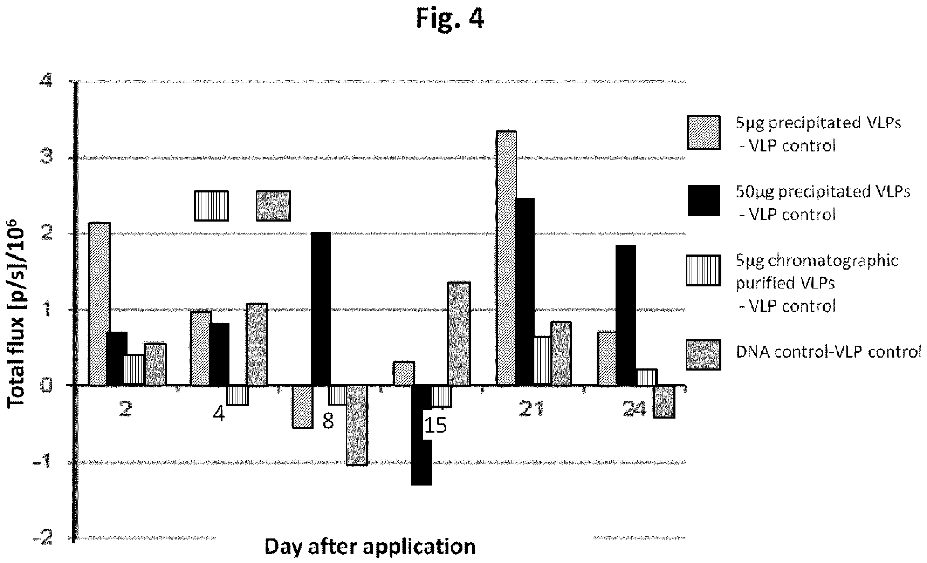

A VP1 according to the present invention may also include a heterologous nuclear localization signal (NLS). Preferably, this NLS is introduced in front of or into the N-terminus of NP1, in particular into the first 30, the first 25, the first 20, the first 15 or the first 10 amino acids of VP1. For example, an NLS as described in WO 2009/036933 (for example page 10, lines 4 to 13 and Figure 4A) or in Shishido-Hara et al. (Shishido-Hara, Y., Hara, Y., Larson, T., Yasui, K., Nagashima, K. & Stoner, G.L. Analysis of Capsid Formation of Human Polyomavirus JC (Tokyo-1 Strain) by a Eukaryotic Expression System: Splicing of Late RNAs, Translation and Nuclear Transport of Major Capsid Protein VP1, and Capsid Assembly, Journal of Virology 74, 1840-1853 (2000).

According to one embodiment, the amino acid sequence according to SEQ ID NO: 5 is introduced into the N-terminal part of VP1, in particular between the amino acids corresponding to amino acids 9 and 10 of SEQ ID NO: 1.

"VP2" or "virus protein 2" according to the present invention refers to a protein which is identical to or is derived from the natural VP2 of the JC virus having the amino acid sequence according to SEQ ID NO: 3. A protein derived from the natural VP2 of the JC virus preferably has an amino acid sequence homology or identity with the amino acid sequence according to SEQ ID NO: 3 of at least 60%, more preferably at least 70%, at least 75%, at least 80%, at least 85%, at least 90%, at least 95%, at least 97%, at least 98% or at least 99% over a sequence of at least 100 contiguous amino acids, preferably at least 150, at least 200, at least 250 or at least 300 contiguous amino acids. Most preferably, the amino acid homology or identity is calculated over the entire length of the natural JCV-VP2. The terms "VP2 derived from the natural VP2 of the JC virus" and "VP2 derived from JC virus" in particular also include VP2 which is identical to the natural VP2 of the JC virus.

The term "VP2" according to the invention also encompasses fractions and derivatives of the natural VP2 which are capable of assembling into VLP together with VP1. Preferably, said fragments of VP2 at least comprise amino acids 214 to 318 of the amino acid sequence according to SEQ ID NO: 3 or a derivative thereof having a homology or identity with the amino acid sequence from amino acid position 214 to 318 of SEQ ID NO: 3 of at least 60%, more preferably at least 70%, at least 75%, at least 80%, at least 85%, at least 90%, at least 95%, at least 97%, at least 98% or at least 99% over a sequence of at least 100 contiguous amino acids, preferably at least 150, at least 200, at least 250 or at least 300 contiguous amino acids, preferably over the entire sequence.

A "peptide" according to the present invention may be composed of any number of amino acids of any type, preferably naturally occurring amino acids, which preferably are linked by peptide bonds. In particular, a peptide comprises at least 3 amino acids, preferably at least 5, at least 7, at least 9, at least 12 or at least 15 amino acids. Furthermore, there is no upper limit for the length of a peptide. However, preferably a peptide according to the invention does not exceed a length of 500 amino acids, preferably 300, 250, 200, 150 or 120 amino acids.

The expression "comprise", as used herein, besides its literal meaning also includes and specifically refers to the expressions "consist essentially of" and "consist of". Thus, the expression "comprise" refers to embodiments wherein the subject-matter which "comprises" specifically listed elements does not comprise further elements as well as embodiments wherein the subject-matter which "comprises" specifically listed elements may and/or indeed does encompass further elements. Likewise, the expression "have" is to be understood as the expression "comprise", also including and specifically referring to the expressions "consist essentially of" and "consist of".

### Administration methods

The VLP of the invention can be administered via various routes. Particularly preferred are dosage forms which allow a systemic effect of the active substance. Most preferred are dosage forms which are administered orally or parenterally, in particular intravenously.

### Manufacturing methods

### Manufacturing of virus-like particles

In a further aspect, the present invention provides a method for producing the virus-like particles according to the present invention. This method in particular comprises the steps of
(a) providing a viral protein VP1 which is derived from JC virus;
(b) optionally providing a viral protein VP2 and or VP3, preferably VP2, which is derived from JC virus and mixing the VP1 with the VP2 (and/or VP3);
(c) allowing the VP1 and optionally the VP2 (and/or VP3) to assembly into virus-like particles.

The method preferably further comprises the step of providing a cargo load and mixing the VP1 and optionally the VP2 and/or VP3 with the cargo load. Preferred is a mixture between VP1 and VP2.

Upon assembly of the VLP, the cargo load preferably is encapsulated inside the VLP. Preferably, at least one VLP carries a cargo load, more preferably at least 1%, at least 2%, at least 3%, at least 5%, at least 7%, at least 10%, at least 15%, at least 20%, at least 25%, at least 30%, at least 40%, at least 50%, at least 60%, at least 70%, at least 80%, at least 90%, at least 95%, at least 97%, at least 98% or at least 99% or 100% of the assembled VLP carry a cargo load.

The assembly of the virus-like particles preferably occurs in solution, more preferably in an aqueous solution. Allowing the assembly of the VLP preferably includes adjusting the Ca²⁺ ion concentration in the solution to a level where assembly of VLP can occur. Said Ca²⁺ ion concentration in particular is in the rage of from 0.1 mM to 5 mM, preferably from 0.2 mM to 3 mM, more preferably from 0.5 mM to 2 mM or from 0.8 mM to 1.2 mM, most preferably about 1 mM. Furthermore, allowing the assembly of the VLP preferably occurs under oxidizing conditions, in particular in the absence of significant concentrations of reducing agents such as DTT, DTE or mercaptoethanol.

Provision of the viral proteins and allowing the VLP assembly may be performed simultaneously. In particular, the viral proteins are provided under conditions where VLP assembly may occur. In preferred embodiments, provision of the viral proteins and VLP assembly is performed in vivo. In particular, the VLP are assembled inside the host cells expressing the viral proteins or upon lysis or disruption of the host cells.

Assembly of the VLP may for example be performed as described in EP 0 862 633, the disclosure of which is herein incorporated by reference.

### Delivery methods

The present invention further provides a method of delivering a substance or composition into a target cell in the central nervous system using the virus-like particles according to the present invention. The method preferably comprises the steps of
(a) providing a vlp according to the present invention which comprises the substance or composition as cargo load; and
(b) administering the virus-like particle into the living body, preferably into a human.

The target cell may be a natural target cell of the JC virus.

The active substance or composition can be of any kind or nature. In a preferred embodiment the active substance is a nucleic acid, in particular a nucleic acid encoding a protein or peptide or an inhibitory nucleic acid such as siRNA or miRNA. In another preferred embodiment the active substance is a protein or peptide. In yet another embodiment of the invention the active substance is a small molecule, in particular a negatively charged small molecule. The active substance can also be a mixture of various active substances.

### Examples

### 1. VP1-VLPs in Blood-Brain-Barrier (BBB) Model in vitro

This *in vitro* experiment shows the ability of the VLPs to cross the BBB in a model system that matches the organisation and properties of the human BBB. In the model system, porcine primary brain endothelial cells (PBCEC) were used which are capable to form the blood-brain-barrier *in vitro* (Angelow S, Zeni P and Galla HJ "Usefulness and limitation of primary cultured porine horoid plexus epithel cells as an in vitro model to study drug transport at the blood-CSF barrier", Adv Drug Delivery 2004; 56(12): 1859-73).

PBCEC preparation and cultivation was conducted as described by Rempe et al., BBRC, 2011: Transport of Poly-(n-butylcyano-acrylate) nanoparticles across the blood-brain-barrier *in vitro* and their influence on barrier integrity.

The effect of cargo-containing VLPs on the PBCEC in Transwell filter system was explored with the help of *relative transendothelial electrical resistance measurement (TEER)* (Rempe *et al.,* 2011). To establish a quantitative proof of the delivery, we used Plasmid DNA as cargo. The integrity of the blood-brain-barrier was not affected by VP1-VLPs under any circumstances and concentrations.

To verify the cargo transport through the blood-brain-barrier *in vitro,* the plasmid DNA was packed into the VLPs as described in Goldmann et al. 1999 (Journal of Virology: Molecular cloning and expression of major structural protein VP1 of the human polyoma virus: formation of virus like particles useful for immunological and therapeutic studies and measured quantitatively by specific qPCR). The copies of plasmid DNA were quantified on the apical and basolateral sides in the BBB *in vitro* model. The apical side is where the VLPs were added (the blood vessel lumen *in vivo*)*,* the basolateral side refers to the brain. The quantitative proof of plasmid DNA on the basolateral side represents the VLP-mediated passage of the molecules through the brain endothelial cells, respectively the cargo delivery through the blood-brain-barrier.

### 2. Reporter gene delivery and expression in vivo

*Reporter gene delivery* experiment were planned to show the capabilities of JC VP1-VLPs to deliver the substances into the cells and organs in the living organism. In this case the reporter gene expression is one of the best methods to demonstrate not only the delivery, but the functionality of the delivered substance too (Hoffman, R.M., 2005: THE MULTIPLE USES OF FLUORESCENT PROTEINS TO VISUALIZE CANCER IN VIVO. Nat. Rev. Cancer; 5(10):796-806).

### Materials

The deferent VP1-VLP probes were tested on the ability to pack and deliver the reporter plasmid into Cos7 (green monkey kidney cells) cells *in vitro, since this cell line is known to be transducible by JCV VLPs.* There were 9 salt precipitated VP1-VLPs probes and 15 chromatographic purified VP1-VLPs probes. The transduction experiments *in vitro* were 5 times repeated. In this experiments, the ability to deliver maximum luminescence signal with the Luciferin substrates for *in vivo* experiments were tested.

Salt precipitated VP1-VLPs were overnight precipitated und dialysed 24 hours against the Standard Buffer (150mM NaCl, 10mM Tris-HCl, pH7,5). The packaging of the Reporter gene plasmid was achieved by chemical dissociation and reassociation as described in Goldmann et al., 1999, Journal of Virology: Molecular cloning and expression of major structural protein VP1 of the human polyoma virus: formation of virus like particles useful for immunological and therapeutic studies.

### The experimental layout

Intravenous injection of VLPs into immunocompetent BALB/c mice was conducted in the tail vein under isoflurane anesthesia.

The animals were grouped as following:
- 5 µg salt precipitated VLPs (4 animals)
- 50 µg salt precipitated VLPs (4 animals)
- 5 µg chromatographic purified VLPs (4 animals)
- DNA control (the reporter gene plasmid only) (3 animals)
- VLPs control (the VP1-VLP capsids only) (3 animals)

The bioluminescence was measured on day 2, 4, 7, 14 and 22, 12 min after intraperitoneal injection of the Luciferin substrate. The results were pooled in each group and the average result and the standard deviation were calculated. The averages were analysed with Two-Way-ANOVA with Holm-Sidak-Test as posthoc-test.

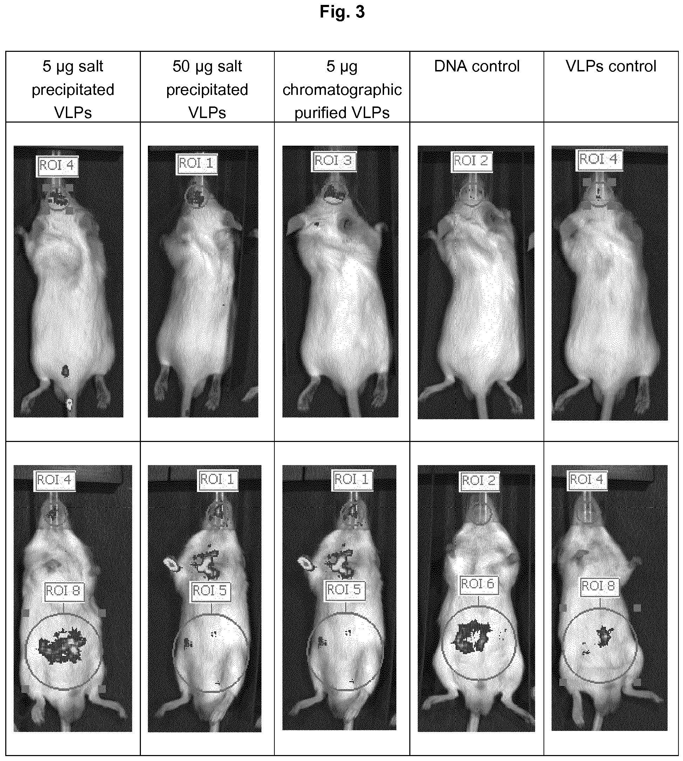

Results are shown in fig. 3 and 4

### 3. Transduction Efficacy of Cos7 Cells (African green monkey kidney cells) with help of the Luciferase plasmids loaded JC VP1-VLPs and JC VP1-VLPs mixed with Luciferase plasmids

1. 18 hours before the Transduction Cos7 Cells were passaged into 24-Well plate.
2. VP1-VLPs were dissociated with DTT and EGTA, mixed with the *Luciferase* plasmid and dialysed against re-association Buffer overnight by +4°C.
3. On the next day, packed VP1-VLPs were taken out of Dialysis.
4. The mixed VP1-VLPs with *Luciferase* plasmid were prepared according to Krauzewicz (Gene Therapy (2000) 7, 1094-1102):
   a. VLPs to *Luciferase* plasmid Ratio mix were 30:1 (w/w)
   b. This mix was incubated 15 min by RT
   c. The mixtures were diluted with DMEM cell media and pipetted to the Cos7 Cells in 24-Well plate.
5. The VP1-VLPs packed with *Luciferase* plasmid were pipetted to the Cos7 Cells; same was conducted with VP1-VLPs mixed with *Luciferase* plasmid.
6. After 72 hours the cells were lysed and Luciferase Activity were measured in Triplets with help of Luciferase Assay from Promega.

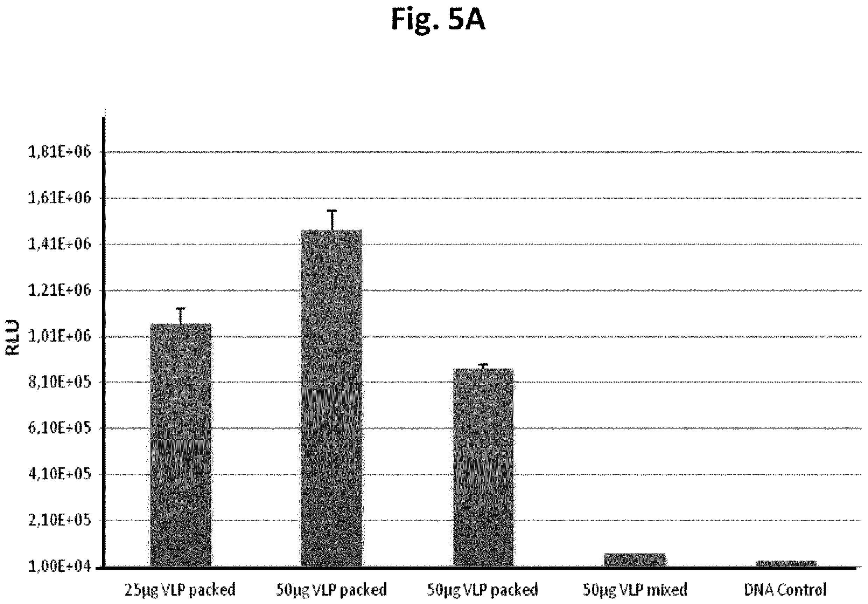

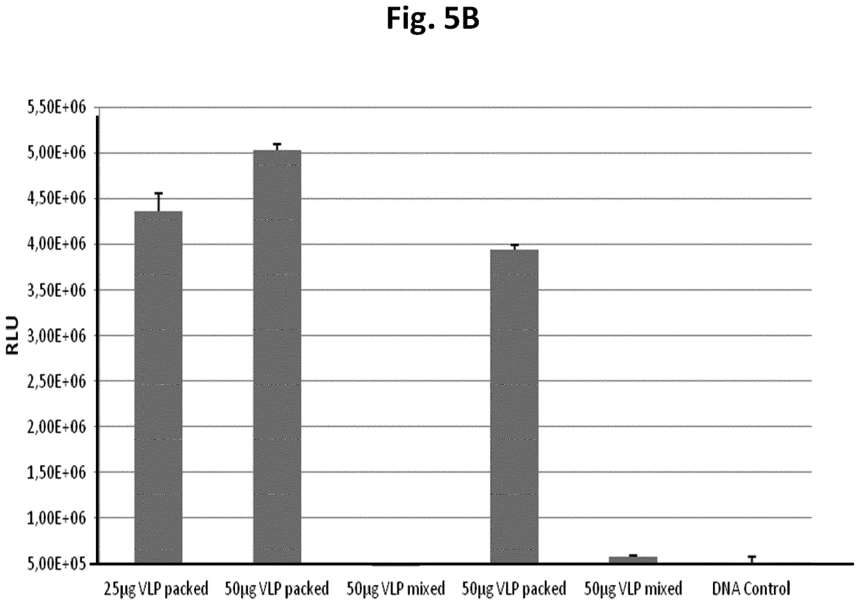

Results of to independent Transduction experiments are shown in fig. 5

### 4. Immunohistochemical detection of Luciferase and VP1 protein in the mouse brain after intravenous application of VLPs packed with a Luciferase plasmid

As showed above, JC VP1-VLPs are capable to deliver substances into cells and organs in the living organism as proved by detection of the plasmid DNA (by qPCR) or the Luciferase activity. These experimental results are further supported by the immunohistochemical detection of the Luciferase protein and VP1 protein in brain cells.

Brain tissues were isolated from mice with an Intravenous injection of VLPs into immunocompetent BALB/c mice (treatment as described above), fixed in PFA and embedded in paraffin as described (J. Jankowski et al., The Journal of comparative Neurology 472: 87-99, 2004: "Engrailed-2 negatively regulates the onset if prenatal Purkinje Cell differentiation"). 7 to 10 µm thin sections were cut and mounted on Histobond plus slides. The sections were re-hydrated, the endogen peroxidase was inactivated and the sections permeabilized. A blocking step was performed in 3% bovine serum albumin solution. Luciferase protein or VP1 protein, respectively were detected with monoclonal antibodies. To increase the signal strength the TSA amplifying fluorescent system (TSA™ Plus Fluorescein System, Perkin Elmer) was used.

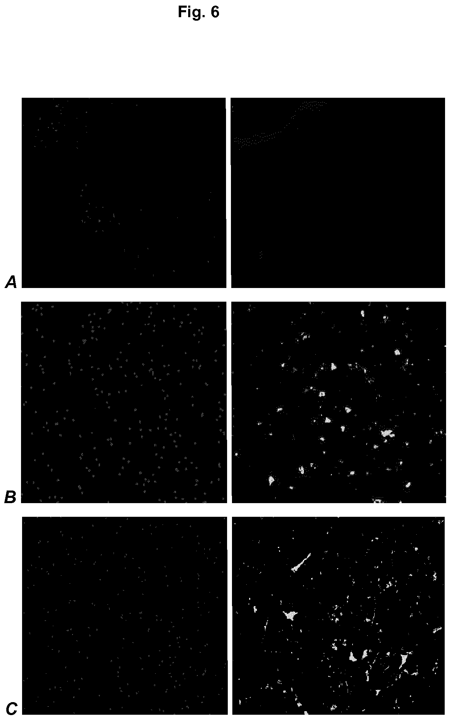

**Results:** As shown in the right panel of Figure 6B, the immunohistochemical analysis using the anti-VP1 antibody were able to detect VP1 protein in cells of the CNS. The brain slice shows scattered spots of irregular size, which is indicative of a cellular localization of VP1 within brain cells. In accordance with the results for the VP1 protein also the Luciferase-derived staining of the brain slice shows scattered spots of irregular size, which is indicative of a cellular localization of Luciferase within brain cells.

**Discussion:** The cellular presence of the luciferase and the VP1 protein in the brain represent a further support of the basic concept of the invention, because it demonstrates that the VLP, and not just the active substance alone, cross the BBB and enter CNS cells.

### 5. Colocalization analysis reveals presence of VP1 protein in oligodendrocytes

Based on the above described cellular detection of VP1 protein in the CNS, a co-localization experiment was performed in order to identify the respective target cells. For this purpose, the detected VP1 protein was co-localized with the marker Olig 2, which is specifically located on oligodendrocytes (B. Menn et al., "Origin of oligodendrocytes in the subventricular zone of the adult brain", The Journal of Neuroscience, 2006, 26(30): 7907-7918).

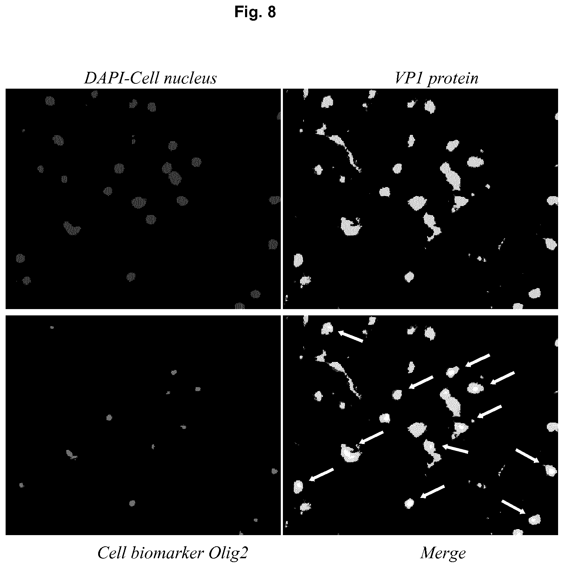

**Results:** As shown in the lower left panel of Figure 8, by using the Olig2 marker several irregular spots could be detected in the brain slice, which in every case are also stained by the nucleus stain DAPI (upper left panel of Figure 8) and therefore indicative of oligodendrocytes. Each of these oligodendrocytes is also positive for VP1 protein as shown in the lower right panel of Figure 8. The cells which are positive for Olig2 and VP1 are marked with a white arrow. Hence, the VP1 protein is localized in CNS oligodendrocytes, which is indicative for a infection of these cells by the intravenously applied VLPs.

**Discussion:** The detection of the VP1 protein in the brain oligodendrocytes is in line with the natural tropism of the JCV virus. As a result the claimed VLPs of the invention are especially suited for a therapeutic treatment of diseases associated with oligodendrocytes such as multiple sclerosis.

### 6. Colocalization analysis reveals presence of VP1 protein and luciferase in neurons

In another co-localization experiment the detected VP1 protein was co-localized with the marker NeuN, which is specifically located on neurons (H. Wolf et al., The Journal of Histochemistry and Cytochemistry, 1996 44(10):1167-1171: "NeuN: A useful neuronal marker for diagnostic histopathology").

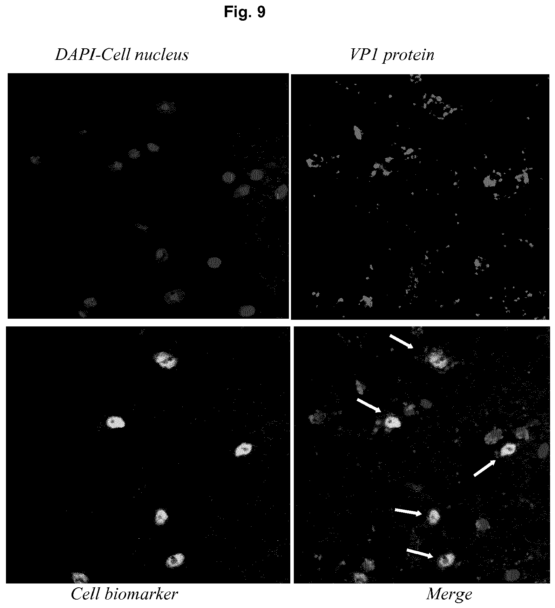

**Results:** As shown in the lower left panel of Figure 9, by using the NeuN marker several irregular spots could be detected in the brain slice, which in every case are also stained by the nucleus stain DAPI (upper left panel of Figure 9) and therefore indicative of neurons. Each of these neurons is also positive for VP1 protein as shown in the lower right panel of Figure 9. The cells which are positive for NeuN and VP1 are marked with a white arrow. Hence, the VP1 protein is localized in CNS neurons, which is indicative for a infection of these cells by the intravenously applied VLPs. The same result, i.e. a colocalization with NeuN was observed for the Luciferase protein (data not shown).

**Discussion:** The detection of the VP1 protein in the brain neurons represent a surprising finding since the natural tropism of the JCV virus is restricted to oligodendrocytes. As a result the claimed VLPs of the invention are especially suited for a therapeutic treatment of diseases associated with neurons such as Parkinson's disease or Alzheimer's disease.

### 7. Colocalization analysis reveals presence of VP1 protein and luciferase in microglia cells

Based on the above described cellular detection of VP1 protein in the CNS, a co-localization experiment was performed in order to identify the respective target cells. For this purpose, the detected VP1 protein was co-localized with the marker Iba1, which is specifically located on microglia cells (D. Ito et al., Molecular brain research, 1998, 57(1): 1-9: "Microglia-specific localization of a novel calcium binding protein, Iba1").

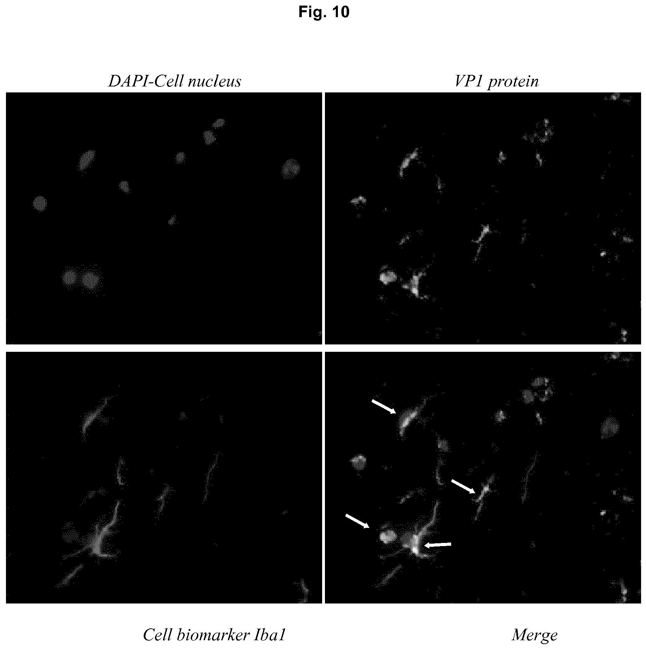

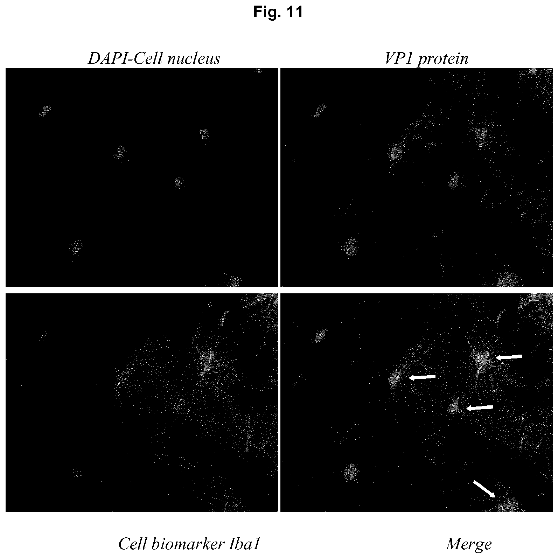

**Results:** As shown in the lower left panel of Figure 9, by using the Iba1 marker several irregular spots could be detected in the brain slice, which in every case are also stained by the nucleus stain DAPI (upper left panel of Figure 10) and therefore indicative of microglia cells. Each of these microglia cells is also positive for VP1 protein as shown in the lower right panel of Figure 10. The cells which are positive for Iba1 and VP1 are marked with a white arrow. Hence, the VP1 protein is localized in CNS microglia cells, which is indicative for a infection of these cells by the intravenously applied VLPs. The same result, i.e. a colocalization with Iba1 was observed for the Luciferase protein (data not shown).

**Discussion:** The detection of the VP1 protein in the brain microglia cells represent a surprising finding since the natural tropism of the JCV virus is restricted to oligodendrocytes. As a result the claimed VLPs of the invention are especially suited for a therapeutic treatment of diseases associated with (activated) microglia cells such as Parkinson's disease or Alzheimer's disease.

### Legend:

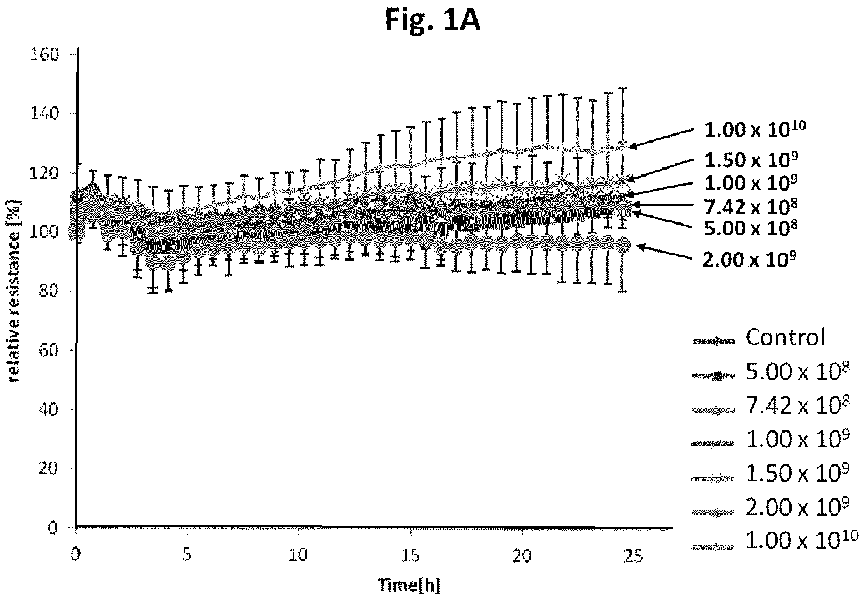

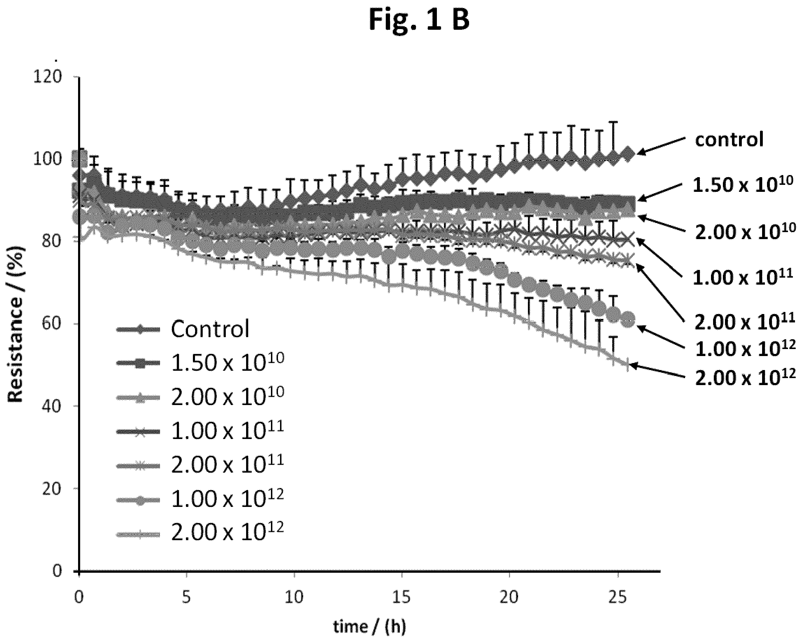

**Fig. 1** **A, B: Relative transendothelial electrical resistance development (A and B)** after addition of the VP1-VLPs in concentrations between 5*10⁸ and 2*10¹² particles per well (n=3).

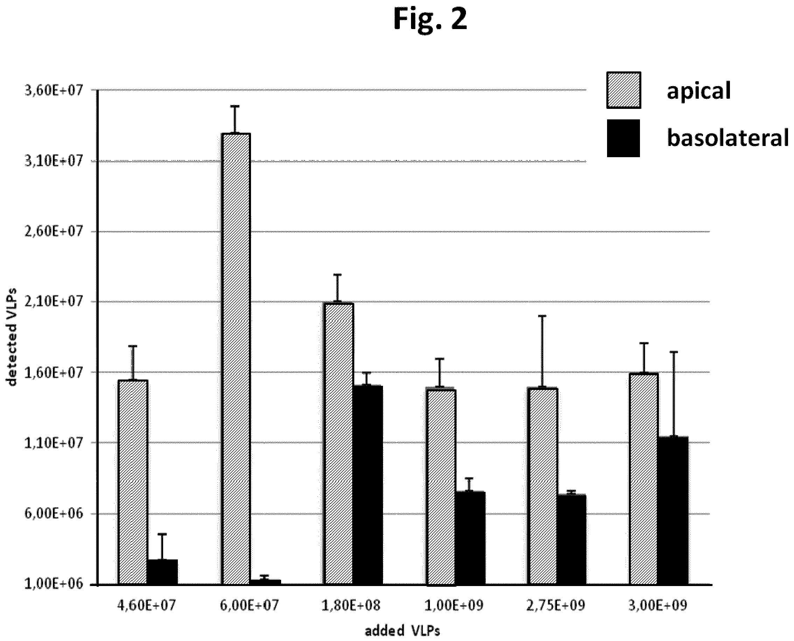

**Fig. 2****: Passage of the VP1-VLPs loaded DNA through the blood-brain-barrier in the *in vitro* model, measured with qPCR with specific primers.** *Added VLPs -* the number of the VLPs added on the apical side in BBB model, *Detected VLPs -* detected number of the plasmid on the apical and basolateral sides in BBB model (n=3)
**Fig. 3****: Bioluminescent signals in mice body after intravenous application:** 5 µg salt precipitated VLPs; 50 µg salt precipitated VLPs; 5 µg chromatographic purified VLPs; DNA control (the reporter gene plasmid only); VLPs control (the VP1-VLP capsids only). As example, 1 mouse of each group on the ventral and dorsal side.
**Fig. 4****: Bioluminescent signals in mice heads after intravenous application:** 5 µg salt precipitated VLPs; 50 µg salt precipitated VLPs; 5 µg chromatographic purified VLPs; DNA control (the reporter gene plasmid only). The average results in the groups minus the VLP control (as a background signal).
**Fig. 5** **A, B: Transduction of the Cos7 cells with help of the VP1-VLPs, loaded with Luciferase plasmid DNA.** RLU -Relative Light Units; 25µg VLP packed - 25µg JC VP1-VLPs packed with *Luciferase* plasmid; 50µg VLP packed - 50µg JC VP1-VLPs packed with *Luciferase* plasmid; 50µg VLP mixed - 50µg JC VP1-VLPs mixed with *Luciferase* plasmid; DNA Control - *Luciferase* plasmid Control.
**Fig. 6****:** Immunohistochemical analysis of brain sections. Left column shows DAPI staining of the cell nucleus, right column the fluorescent labeled proteins: A- negative control; B- FITC-fluorescent staining of VP1 protein; C- FITC-fluorescent staining of Luciferase protein.

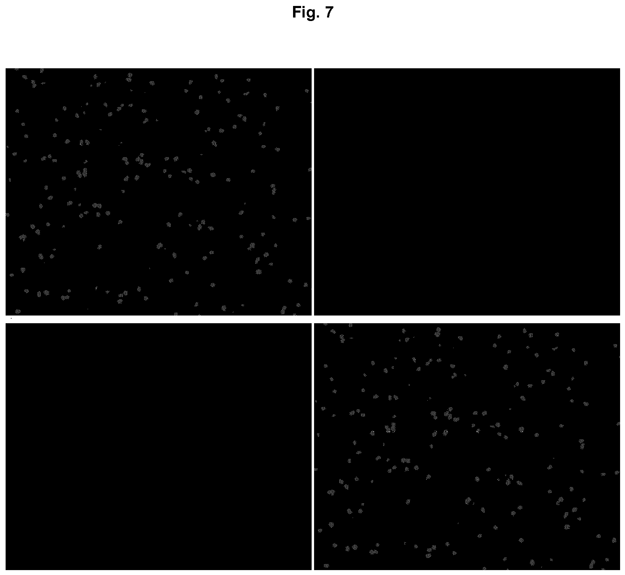

**Fig. 7****:** Negative controls for the co-localization of VP1 protein with the oligodendrocyte marker Olig2. Upper left panel shows staining of the cell nuclei with the dye DAPI. Upper right panel shows signal after fluorescence detection without the use of the anti-VP1 antibody. Lower left panel shows signal after fluorescence detection without the use of the anti-Olig2 antibody. Lower right panel represents the merged pictures of the two control stains.
**Fig. 8****: Co-localization of VP1 protein with the oligodendrocyte marker Olig2.** Upper left panel shows staining of the cell nuclei with the dye DAPI. Upper right panel shows the localization of the VP1 protein (FITC-fluorescence). Lower left panel shows the staining with the anti-Olig2 antibody (TRITC-fluorescence). Lower right panel represents the merged pictures of the staining for the Olig2-marker and the VP1 protein. Cells which are positive for VP1 and Olig2 are marked with an arrow.
**Fig. 9****: Co-localization of VP1 protein with the neuronal marker NeuN.** Upper left panel shows staining of the cell nuclei with the dye DAPI. Upper right panel shows the localization of the VP1 protein (FITC-fluorescence). Lower left panel shows the staining with the anti-NeuN antibody (TRITC-fluorescence). Lower right panel represents the merged pictures of the staining for the NeuN-marker and the VP1 protein. Cells which are positive for VP1 and NeuN are marked with an arrow.
**Fig. 10****: Co-localization of VP1 protein with the microglia marker Iba1.** Upper left panel shows staining of the cell nuclei with the dye DAPI. Upper right panel shows the localization of the VP1 protein (FITC-fluorescence). Lower left panel shows the staining with the anti-Iba1 antibody (TRITC-fluorescence). Lower right panel represents the merged pictures of the staining for the Iba1-marker and the VP1 protein. Cells which are positive for VP1 and Iba1 are marked with an arrow.

### SPECIAL EMBODIMENTS

1. VLP derived from human polyoma virus for use as a drug delivery system, wherein the drug is transported to and/or into microglia cells or neurons of the CNS.
2. VLP according claim 2, wherein said VLP is derived from JCV.
3. VLP according to claim 1 or 2, wherein said drug delivery system is used for the treatment or diagnosis of neurological disorders.
4. VLP according to claim 3 wherein the neurological disorder is selected from the group consisting of stroke, alcohol addiction, Alzheimer Disease, anxiety, arthritis, asthenia, attention deficit hyperactivity disorder, bipolar disorder, cancer pain, cerebral ischemia, cerebral neuroprotectant, cervical dystonia, Chorea associated with Huntington's disease, chronic pain, chronic severe pain, cognitive disorder, cortical myoclonus, degenerative Nerve Diseases, depression, diabetic neuropathic pain, diabetic neuropathy, emotional lability, epilepsy, excessive sleepiness associated with narcolepsy, fibromyalgia, Fragile X syndrome, Friedreich's ataxia, insomnia, Lennox Gastaut syndrome, major depressive and anxiety disorders, manic episodes associated with bipolar disorder, memory impairment, migraine, mild cognitive impairment, moderate to severe pain, motor neuron disease, multiple sclerosis, musculoskeletal pain, narcolepsy, neuralgia, neuropathic pain, nicotine dependence, obsessive compulsive disorder, opioid-induced adverse effect, opioid-induced constipation, osteoarthritis pain, overactive bladder, pain, Parkinson's disease, pediatric drooling, peripheral diabetic neuropathy, post-operative pain, postherpetic neuralgia, premenstrual dysphoric disorder, psychosis, refractory complex partial seizures, restless leg syndrome (RLS), schizophrenia, seizure, severe chronic pain, sleep disorder, smoking cessation, spasticity, spinal cord injury, stroke, transthyretin familial amyloid polyneuropathy, traumatic brain injury, vertigo, cachexia, amyotrophic lateral sclerosis, spinocerebellar ataxia type I, extrapyramidal and movement disorders, transient ischemic attack (TIA), Progressive multifocal leukoencephalopathy (PML), HIV-infection, dementia, such as Alzheimer's disease, vascular dementia, frontotemporal dementia, semantic dementia and dementia with Lewy bodies.
5. VLP according claim 3, wherein the neurological disorder is Alzheimer's disease.
6. VLP according claim 3, wherein the neurological disorder is Parkinson's disease.
7. VLP according claim 3, wherein the neurological disorder is multiple sclerosis.
8. VLP according to at least one of the above claims, wherein said VLP enters said CNS cell together with the drug encapsulated by the VLP.
9. VLP according to at least one of the above claims, wherein that drug delivery system crosses the physiologically intact blood-brain barrier.
10. VLP according to at least one of the above claims, wherein the VLP is composed of VP1 proteins of JC virus.
11. VLP according to at least one of the above claims, wherein the VLP is prepared for oral, parenteral or intravenous administration and in particular for intravenous administration.
12. VLP according to at least one of the above claims, wherein the VP1 comprises an amino acid sequence which is at least 80% identical to the amino acid sequence according to SEQ ID NO: 1 over its entire length and/or wherein the VP2 comprises an amino acid sequence which is at least 80% identical to the amino acid sequence according to SEQ ID NO: 3 over its entire length.
13. VLP according to any one of the above claims, wherein the drug is selected from the group consisting of a CNS active compound, a detectable agent such as a radionuclide, a protein, a peptide and a nucleic acid, in particular selected from the group consisting of nucleic acids encoding a desired protein such as mRNA, cDNA, a plasmid or vector; inhibitory nucleic acids such as siRNA or miRNA; and nucleic acids having catalytic activity such as a ribozyme.
14. VLP according to claim 13, wherein the drug is selected from the group consisting of monoclonal antibodies, antipsychotic drugs, analgesic drugs, thrombolytics, antidepressants, immunmodulators, immunosuppressants, acetylcholinesterase inhibitors, glutamate receptor antagonists or modulators, such as NMDA receptor antagonists, psychostimulants, anti-dementia drugs, anxiolytic drugs, nootropic drugs, metabolic enhancers, metabolic modulators, neuroprotective drugs, and anticonsulvants.
15. Pharmaceutical composition comprising a VLP according to any of the above claims wherein at least 1%, preferably at least 15%, more preferably at least 50%, in particular preferred at least 95% of the total amount of drug substance (cargo) is fully encapsulated in the hull of the VLP.
16. Pharmaceutical composition according to claim 15 wherein the VLPs are non-aggregated.

## Claims

1. Use of a VLP derived from human polyoma virus comprising a drug as a drug delivery system for the drug delivery to and/or into a microglia cell and/or a neuron.

2. The use according to claim 1, wherein the VLP is derived from a human polyoma virus comprising Human polyomavirus 6 (HPyV6), Human polyomavirus 7 (HPyV7), Human polyomavirus 9 (HPyV9), BK polyomavirus (BKPyV), JC polyomavirus (JCPyV), Merkel Cell polyomavirus (MCPyV), KI polyomavirus (formerly known as Karolinska Institute polyomavirus, KIPyV), WU polyomavirus (formerly known as Washington University polyomavirus, (WUPyV), Trichodysplasia spinulosa-associated polyomavirus (TSV), human polyoma virus 10 (HPyV10), MW polyomavirus and MX polyomavirus.

3. The use according to claim 1 or 2, wherein the VLP is derived from JCV.

4. The use according to any of the preceding claims for the drug delivery to a surface of the microglia cell and/or the neuron.

5. The use according to any of the preceding claims for the drug delivery with or without the VLP across a plasma membrane of the microglia cell and/or the neuron into a cytoplasma, an endosome, or a nucleus.

6. The use according to any of the preceding claims, wherein the microglia cell and/or the neuron are located in a spinal cord or in a brain, in particular in the brain.

7. The use according to any of the preceding claims, wherein the VLP comprises at least one VP1.

8. The use according to claim 7, wherein the VLP additionally comprises at least one VP2 and/or at least one VP3.

9. The use according to claim 7 or 8, wherein the VP1 comprises an amino acid sequence which is at least 80% identical to the amino acid sequence according to SEQ ID NO: 1 over its entire length and/or wherein the VP2 comprises an amino acid sequence which is at least 80% identical to the amino acid sequence according to SEQ ID NO: 3 over its entire length.

10. The use according to any of claims 7 to 9, wherein the at least one VP1 and/or the at least one VP2 and/or the at least one VP3 is modified by forming a fusion protein, or post-translationally or chemically modified.

11. The use according to any of the preceding claims, wherein the VLP further comprises at least one heterologous protein.

12. The use according to any of the preceding claims, wherein the drug is selected from the group consisting of a CNS active compound, a detectable agent such as a radionuclide, a protein, a peptide and a nucleic acid, in particular selected from the group consisting of nucleic acids encoding a desired protein such as mRNA, cDNA, a plasmid or vector; inhibitory nucleic acids such as siRNA or miRNA; and nucleic acids having catalytic activity such as a ribozyme.

13. The use according to any of the preceding claims, wherein the drug is selected from the group consisting of monoclonal antibodies, antipsychotic drugs, analgesic drugs, thrombolytics, antidepressants, immunmodulators, immunosuppressants, acetylcholinesterase inhibitors, glutamate receptor antagonists or modulators, such as NMDA receptor antagonists, psychostimulants, anti-dementia drugs, anxiolytic drugs, nootropic drugs, metabolic enhancers, metabolic modulators, neuroprotective drugs, and anticonsulvants.

14. The use according to any of the preceding claims, wherein the drug delivery system is cell-free.
